(19) Europäisches Patentamt
European Patent Office
Office européen des brevets

(11) **EP 4 076 288 B1**

(12) **EUROPEAN PATENT SPECIFICATION**

(45) Date of publication and mention
of the grant of the patent:
**30.10.2024 Bulletin 2024/44**

(21) Application number: **20834094.3**

(22) Date of filing: **09.12.2020**

(51) International Patent Classification (IPC):
**A61F 2/28** *(2006.01)* **A61F 2/30** *(2006.01)*
**A61L 31/06** *(2006.01)*

(52) Cooperative Patent Classification (CPC):
(C-Sets available)
**A61F 2/2875; A61F 2/3094; A61L 31/026;**
**A61L 31/06; A61L 31/146; A61L 31/148;**
A61F 2002/30062; A61F 2002/30113;
A61F 2002/30224; A61F 2002/30233;
A61F 2002/30354; A61F 2002/30405;
A61F 2002/30914; A61F 2002/30915;
A61F 2002/30971; (Cont.)

(86) International application number:
**PCT/US2020/064102**

(87) International publication number:
**WO 2021/126638 (24.06.2021 Gazette 2021/25)**

(54) **RESORBABLE IMPLANTS FOR RECONSTRUCTION OF BONE DEFECTS**

RESORBIERBARE IMPLANTATE ZUR REKONSTRUKTION VON KNOCHENDEFEKTEN

IMPLANT RÉSORBABLES POUR LA RECONSTRUCTION DE DÉFAUTS OSSEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2019 US 201962951921 P**

(43) Date of publication of application:
**26.10.2022 Bulletin 2022/43**

(73) Proprietor: **Tepha, Inc.**
**Lexington MA 02421 (US)**

(72) Inventors:
• **SARIIBRAHIMOGLU, Kemal**
**Lexington, Massachusetts 02421 (US)**
• **LIMEM, Skander**
**Lexington, Massachusetts 02421 (US)**
• **GANATRA, Amit**
**Lexington, Massachusetts 02421 (US)**
• **RIZK, Said**
**Lexington, Massachusetts 02421 (US)**
• **WILLIAMS, Simon, F.**
**Lexington, Massachusetts 02421 (US)**

(74) Representative: **Hoffmann Eitle**
**Patent- und Rechtsanwälte PartmbB**
**Arabellastraße 30**
**81925 München (DE)**

(56) References cited:
WO-A1-01/30250 US-A1- 2009 317 447
US-A1- 2013 053 900 US-A1- 2015 150 681

(52) Cooperative Patent Classification (CPC): (Cont.)
A61F 2002/30985; A61L 2430/02

C-Sets
**A61L 31/06, C08L 67/04**

**Description**

FIELD OF THE INVENTION

The present invention generally relates to the field of surgery, and more particularly, to implantable medical devices for bone reconstruction, the repair of bone defects, and cranium reconstruction.

BACKGROUND OF THE INVENTION

Head injuries resulting in damage to blood vessels in the meninges, a thin layer of tissues surrounding the brain, can cause an acute or chronic subdural hematoma (CSDH). CSDH occurs when one of the blood vessels in the meninges is ruptured, and blood builds up causing a swelling just below the dura mater, the outermost tissue of the meninges. CSDH is a dangerous condition needing immediate intervention because the swelling of blood, which has nowhere to go, can compress the brain, and can result in death.

Neurosurgeons treat CSDH by making small holes, called burr holes, in the cranium. These holes relieve pressure on the brain caused by the accumulation of blood. The process of creating the small hole in the cranium is known as trephination.

Treatment of CSDH is rapidly becoming one of the most common neurosurgical procedures not least because of an aging population and the increased use of antithrombotic drugs.

Neurosurgeons may sometimes also treat epidural hematomas, caused by a buildup of blood beneath the cranium and just above the dura layer, in the same manner. Epidural hematomas tend to be more common in younger patients. Neurosurgeons may also make burr holes in order to treat other conditions, such as certain kinds of brain cancer, hydrocephalus, bleeding from the brain, and the accumulation of pus around the meninges.

Patients with conditions such as CSDH often make a full neurological recovery following surgery, however, defects in the cranium caused by burr hole trephinations frequently result in undesirable depressions in the scalp. These depressions are often not only cosmetically unacceptable to the patient, but can be troublesome during combing and hairdressing.

Attempts to overcome the problems associated with bone defects or depressions have included the use of various covers and plugs to obtain a more acceptable cosmetic and functional result. Covers and plugs also serve to seal the burr holes protecting the underlying tissues and brain from infection or other potential injuries.

A common option for repairing burr holes has been the use of covers that are fixated over the top of the burr hole. These covers are typically screwed into the cranium. The covers are often made from metals, such as titanium, and are typically in the form of plates with holes for fixation. They are not ideal, however, because a permanent foreign body is left in the patient.

As an alternative to permanent titanium covers, burr holes have been packed with absorbable gelatin sponges, for example, GELFOAM, sold by Pfizer. Im et al. (The efficacy of titanium burr hole cover for reconstruction of skull defect after burr hole trephination of chronic subdural hematoma, Korean J Neurotrauma, 2014, 10(2):76-81) compared the efficacy of packing GELFOAM into a burr hole to the use of a titanium burr hole cover. On follow up, 40% of patients treated with titanium burr hole covers were dissatisfied with the cosmetic result, and 40% of patients complained of functional handicaps. In comparison, 76.6% of the GELFOAM patients interviewed were dissatisfied with the cosmetic result, and 64.1% of patients complained of functional handicaps. Patients treated with GELFOAM were reported to have a mean scalp depression of 2.45 mm following treatment.

Other approaches to filling burr holes have included the use of autologous tissues, such as bone, bone dust, muscle, and fat tissue which all require harvesting, and the use of synthetic substitutes, such as polymethyl methacrylate or polypropylene, which are permanent materials, and polycaprolactone. Kubota et al. (Long-term follow-up for ossification of autologous bone plug and skin sinking after periosteum-preserved burr hole surgery, Surgical Neurology International, 2017, 8:204.) disclose the use of bone plugs made from bone dust. The degree of skin sinking at 12 months for patients receiving the bone plugs was reported to be an average of 1.2 mm with the range as high as 2.35 mm.

US Patent No. 20070083268 to Teoh et al. discloses plugs made from polycaprolactone to repair burr holes. Schantz et al. 2006 (Cranioplasty after trephination using a novel biodegradable burr hole cover: technical case report, Neurosurgery, 2006, 58(1 Suppl): ONS-E176) also discloses the use of bone plugs comprising polycaprolactone.

US Patent No. 6,350,284 to Törmälä et al. discloses a bioabsorbable cranial implant with a rigid plate layer and fibrous web layer for attaching to the outer skull of a patient to repair a defect in the cranium.

US Patent 2015150681 to Ricci et al. discloses a tissue repair device or scaffold having a porous bone ingrowth structure containing interconnected struts surrounded by a microporous shell.

Notwithstanding the above, there is still a need for devices as described herein that can be used to repair burr holes. In particular, there is a need to develop devices to repair burr holes that prevent the formation of depressions in the cranium following surgery. Such devices would provide an improved cosmetic result for the patient, and also eliminate functional handicaps that can arise, for example, during combing and hairdressing. These devices would also be easy to implant, and they would not interfere with any subsequent imaging techniques, such as CT and magnetic resonance imaging. There is also a need to develop devices to repair burr holes that would not leave permanent foreign bodies in the cranium, but rather facilitate regeneration of calvarial bone so that the properties of the regenerated bone mimic the properties of the hard tissue surrounding the burr hole. Ideally, the devices would allow an early and stable integration in the cranium, and they would be replaced over time as they are resorbed with autologous bone. There is also a further need to develop devices for closure of burr holes that allow re-access to the burr hole for a subsequent procedure without requiring the surgeon to drill another burr hole in the patient's cranium.

SUMMARY OF THE INVENTION

A device to repair a bone defect according to the invention is defined in claim 1. The device comprises a stem connected to a cap with a flange. The device further comprises bristles emanating from the circumference of the stem. The device is porous.

Medical devices are described herein that can be used in the reconstruction of the cranium, particularly following surgical procedures where a neurosurgeon creates a burr hole in the patient's cranium, and the device is used to repair the burr hole. In embodiments, the devices are located substantially in the burr hole, or a component of the device is located in the burr hole and a component of the device is located outside of the burr hole on the outer surface of the cranium. The component on the outer surface of the cranium prevents an implanted device from being dislodged from the burr hole, and ending up, for example, on the inside of the cranium between the cranium and meninges. In embodiments, the devices do not extend below the thickness of the cranium (i.e. do not extend into the space between the cranium and meninges).

The devices have stem and cap components, and the cap is connected to the stem. In a sense, the stem is a boss protruding from the cap. The stem component is inserted in the burr hole when the device is implanted, with the cap component located on the outer surface of the cranium. The cap component has a flange that ensures that the stem of the device cannot be dislodged from the burr hole, or jut out beneath the cranium.

The medical device comprises filament elements that extend beyond the circumference of the stem. The filament elements provide the device with a self-locking feature. The filament elements can engage cancellous bone of the cranium to secure the device in a burr hole. In embodiments, the filament elements are flexible bristles.

In some embodiments, barbs, hooks or tines extend from the stem body to engage with the wall of the bone hole, and prohibit removal of the medical device.

In embodiments, the cap has a convex shape. The convex shape is designed to reduce the formation of depressions in the cranium at the burr hole site.

In embodiments, the devices have stems that apply pressure to the side walls of the burr holes when the stem is inserted in a burr hole. In embodiments, the stems are designed to expand within the burr holes. In embodiments, the stems are designed to be compressed within the burr holes.

The device comprises a stem component connected to a cap component. In embodiments, the stem of the device is sized to at least partly fill the burr hole, and more preferably is sized to fill the burr hole. The cap of the device has a flange that is sized to prevent the device from being dislodged, and to prevent the stem of the device from being pushed too far through the burr hole towards the meninges. In embodiments, the devices are sized so that the difference in diameter of the stem of the device that is inserted in the burr hole and the diameter of the burr hole is not more than 2 mm or 1 mm, preferably wherein the diameter of the stem is less than the diameter of the burr hole. In embodiments, the diameter of the stem of the device increases after implantation resulting in a tight fit of the device in the burr hole, and a tight seal.

In other embodiments, the devices are rivets that comprise two separate components, a pin component and a cylindrical hollow core component with a flange. The cylindrical hollow core component is inserted into a burr hole until the flange abuts the outer surface of the cranium. The pin component is then inserted inside the cylindrical hollow core component causing expansion of the cylindrical hollow core component and compression of the cylindrical hollow core component against the side walls of the burr hole. In embodiments, the pin component is a rivet push pin wherein the length of the pin decreases when it is inserted in the cylindrical hollow core component, and its diameter increases. In other embodiments, the devices comprise a pin component and a cylindrical hollow core component with a flange, and the pin is threaded so that it can be screwed into the cylindrical hollow core component. Screwing the pin into the cylindrical hollow core component results in the cylindrical hollow core component expanding, and applying pressure to the side walls of the burr hole to anchor the device in place and seal the burr hole. In embodiments, the pins are made of permanent materials, such as permanent polymers, so that they can be removed for subsequent procedures.

In embodiments, the device comprises a cylindrical stem component connected to a cap with a flange.

The stem and cap have a porous structure. In some embodiments, the stem and cap have an open porous structure.

In embodiments, the device comprises a triangular open pore structure, and the triangular open pore structure comprises layers of crisscrossed filaments. In embodiments, the triangular open pore structure is formed with layers of filaments positioned at angles of 0, 60 and 120° to each other. In embodiments, the device with the triangular open pore structure has an elastic modulus of 0.5 MPa to 20 GPa.

In embodiments, the cylindrical stem component is sized to be inserted in a bone defect, and the flange has a diameter larger than the diameter of the cylindrical stem.

In embodiments, the device with the triangular open pore structure comprises a resorbable polymer. In embodiments, the resorbable polymer is poly-4-hydroxybutyrate or copolymer thereof, or poly(butylene succinate) or copolymer thereof.

In embodiments, burr holes are sealed by physical expansion of a medical device inside the burr hole. The device preferably further comprises a flange to correctly locate the device in the burr hole. In other embodiments, the devices are secured in place using fibrin glue.

The devices reduce or prevent the formation of depressions in the cranium following surgeries where burr holes are formed. The devices produce an improved cosmetic result with no depressions or only slight depressions forming in the cranium post-operatively after implantation of the devices. The devices reduce or eliminate functional handicaps that can result, for example, from combing and hairdressing.

In embodiments, the devices create a hard tissue at the entry point to the burr hole on the outer surface of the cranium after implantation. The hard tissue prevents the repair site from being palpable, and from depressions forming at this location. In embodiments, the devices create a hard tissue at the entry point to the burr hole on the outside surface of the cranium, and a softer tissue within the burr hole.

The devices are preferably resorbable unless they comprise a permanent pin, and degrade after implantation. In embodiments, the devices are sized to at least partly fill the burr hole, and more preferably incorporate the physical shape of the burr hole.

In embodiments, the devices are implanted without fixation of the device to the outer surface of the cranium. The devices are implanted without the use of additional anchoring devices such as screws, tacks, staples, and sutures.

The devices preferably degrade over time leaving no trace of the device. The devices are rapidly and stably integrated into the cranium. The devices are designed to allow tissue in-growth, and specifically bone ingrowth in the burr hole. The device preferably induces osteogenesis and the formation of new bone in the burr hole as the device degrades, and more preferably the burr hole is filled with calvarial bone. The properties of the new bone formed in the burr hole preferably have similar properties to the properties of the bone surrounding the burr hole. The devices produce a stable cranioplasty. The devices preferably do not leave any permanent foreign bodies in the cranium unless they comprise a permanent pin that can be removed in subsequent procedures. The devices allow burr holes to be filled without depressions forming on the outer surface of the cranium. The repaired burr hole is stable to palpation. Palpation at 3, 6 and 12 months follow-up post-surgery preferably does not reveal any depressions at the burr hole site.

The devices are porous, and more preferably have a porosity of 50-75%, or a filling density of 0.4 to 0.7. In embodiments, the devices have different porosities in different components of the device. In embodiments, the devices have high porosity in the region of the diploë of the cranium, and lower porosity in the region located at the entry point to the burr hole. In embodiments, the devices have densities of 0.2 to 0.6 g/cm$^3$.

In embodiments, the porosity of the stem component is different to the porosity in the cap component for devices having stem and cap components. In embodiments, the pore dimensions in the stem component may be different when measured in the longitudinal direction (along the axis of the stem) versus the transverse direction (along the cross section of the stem). In embodiments, the pore dimensions in the cap component may be different when measured in the longitudinal direction (in the direction of the axis of the device) versus the transverse direction (along the cross section of the cap). In embodiments, the pores in the stem component have dimensions of 0.05 to 2 mm in the longitudinal direction. In embodiments, the pores in the stem component have dimensions of 0.05 to 1 mm in the transverse direction. In embodiments, the pores in the stem component have dimensions of 0.05 to 2 mm in the longitudinal direction, and dimensions of 0.05 to 1 mm in the transverse direction, wherein the dimensions in the longitudinal direction are selected to be greater than the dimensions in the transverse direction. In embodiments, the pores in the cap component have dimensions of 0.01 to 0.5 mm in the longitudinal direction. In embodiments, the pores in the cap component have dimensions of 0.01 to 0.2 mm in the transverse direction. In embodiments, the pores in the cap component have dimensions of 0.01 to 0.5 mm in the longitudinal direction, and dimensions of 0.01 to 0.2 mm in the transverse direction, wherein the dimensions in the longitudinal direction are selected to be greater than the dimensions in the transverse direction.

In embodiments, the devices comprise filaments, including 3D printed filaments, and the thickness of the filaments are 0.05 to 0.8 mm.

In embodiments, the devices have an elastic modulus of 0.5 MPa to 20 GPa, more preferably 1 MPa to 4 GPa, and even more preferably 10 MPa to 1 GPa.

In embodiments, the devices further comprise a ceramic. The ceramic helps to promote bone formation in the implant. In embodiments, the ceramics include hydroxyapatites, α-tricalcium phosphate, β-tricalcium phosphate (β-TCP), sintered hydroxyapatite, and precipitated hydroxyapatite, monocalcium phosphate monohydrate, dicalcium phosphate dihydrate, dicalcium phosphate anhydrous, amorphous calcium phosphate, tetracalcium phosphate, and octacalcium phosphate. In embodiments, the devices have different amounts of ceramic in different components or locations of the device. In embodiments, the amount of ceramic in the stem component of the device is different from the amount of ceramic in the cap component of the device for devices comprising stem and cap components. In embodiments, the amount of ceramic in the stem component is less than the amount of ceramic in the cap component. In embodiments, the amount of ceramic in the stem component is 0.5 to 50 wt. %. In embodiments, the amount of ceramic in the cap component is 0.5 to 80 wt. %, and more preferably 5 to 50 wt. %. In embodiments, the amount of ceramic in the stem component is 0.5 to 50 wt. %, and the amount of ceramic in the cap component is 1 to 70 wt. %, wherein the amount of ceramic in the stem is less than the amount of ceramic in the cap. In embodiments, the amount of ceramic in the implanted device is greater at the entry point to the burr hole than the amount of ceramic within the region of the diploë (the cancellous bone located between the external and inner layers of compact bone of the cranium). Increased amounts of ceramic in the implanted device in the vicinity of the entry point to the burr hole promotes the formation of hard tissue at the entry point that cannot be depressed. The use of high percentages of ceramic at this burr hole entry point location helps to prevent the formation of soft tissue in this vicinity, and to prevent the formation of palpable tissue and depressions in the cranium. Lower percentages of ceramic in the implanted device in the diploë region helps to create a slightly softer and more porous cancellous bone structure.

In embodiments, the devices comprise filaments, including 3D printed filaments, and the thickness of the filaments are 0.05 to 0.8 mm.

Methods to prepare the devices are also described. The devices are preferably made using 3D printing or molding, including injection molding, but may also be prepared by other methods including particle leaching, phase separation, foaming, and fiber processing. The devices are preferably made by 3D printing or molding a composition comprising a ceramic and P4HB or copolymer thereof, or PBS or copolymer thereof, to produce a device comprising a stem for insertion in a burr hole with a connected cap, or a device comprising a cylindrical hollow core component with a flange and a separate pin that is inserted into the cylindrical hollow core component.

In embodiments, the devices comprising ceramic are prepared so that the amount of ceramic in the vicinity of the entry point to the burr hole when the device is implanted, is greater than the amount of ceramic located within the center of the burr hole. Preferably, the ceramic is β-TCP.

The devices may be 3D printed, and have structures comprising filaments. Preferably, the 3D printed devices are printed using melt extrusion deposition. In embodiments, the devices are 3D printed to have filaments wherein the thicknesses of the filaments are 0.05 to 0.8 mm. The distances between the filaments in the 3D printed device may vary. The distances between filaments in the stem component of a device may be different to the distances between filaments in the cap of a device for a device comprising a cap and a stem. In embodiments, the distance between filaments in the stem component of a device are 0.05 to 2 mm. In embodiments, the distance between filaments in the cap component of a device are 0.01 to 0.5 mm. In embodiments, the distance between filaments in the stem component of a device are 0.05 to 2 mm, and the distance between filaments in the cap component of a device are 0.01 to 0.5 mm, wherein the distances between filaments in the stem component of the device are greater than the distances between filaments in the cap component of the device, and optionally wherein the thicknesses of the filaments are 0.05 to 0.8 mm.

In embodiments, the pores in the stem component of a device comprising cap and stem components have dimensions of 0.05 to 2 mm in the longitudinal direction. In embodiments, the pores in the stem component of a device comprising cap and stem components have dimensions of 0.05 to 1 mm in the transverse direction. In embodiments, the pores in the stem component of a device comprising cap and stem components have dimensions of 0.05 to 2 mm in the longitudinal direction, and dimensions of 0.05 to 1 mm in the transverse direction, wherein the dimensions in the longitudinal direction are selected to be greater than the dimensions in the transverse direction. In embodiments, the pores in the cap component of a device comprising cap and stem components have dimensions of 0.01 to 0.5 mm in the longitudinal direction. In embodiments, the pores in the cap component of a device comprising cap and stem components have dimensions of 0.01 to 0.2 mm in the transverse direction. In embodiments, the pores in the cap component of a device comprising cap and stem components have dimensions of 0.01 to 0.5 mm in the longitudinal direction, and dimensions of 0.01 to 0.2 mm in the transverse direction, wherein the dimensions in the longitudinal direction are selected to be greater than the dimensions in the transverse direction.

The devices comprise cap and stem components with filament elements emanating from the circumference of the stem component. In embodiments, the protruding filament elements have lengths, measured from the circumference of the stem component to the tip of the element, of 0.1 to 5 mm, and diameters of 0.15 to 0.8 mm.

The devices are preferably formed from resorbable polymers. The resorbable polymers include poly-4-hydroxybutyrate (P4HB) and copolymers thereof, and poly(butylene succinate) (PBS) and copolymers thereof. In embodiments, the devices are formed from poly-4-hydroxybutyrate and copolymers thereof comprising one or more ceramics, or poly(butylene succinate) or copolymers thereof comprising one or more ceramics.

In embodiments, the devices further comprise bioactive agents. In embodiments, the devices comprise antimicrobials or antibiotics.

The devices comprise materials that do not interfere with imaging techniques, including CT and magnetic resonance imaging.

In embodiments, the device can be used in the repair of metaphyseal bone defects resulting, for example, from the removal of cysts, tumors, and orthopedic devices, as well as from the harvesting of autograft. In other embodiments, the device can be used in the repair of traumatic fractures, including fractures of the distal radius, proximal humerus, pelvic bone, proximal femur, distal femur, tibial plateau, tibial pilon, and the calcaneus. The device may be used to repair bone defects resulting from the revision of total joints, or osteotomy procedures, for example, of the distal radius or tibial plateau. The devices may also be used in the treatment of tuberosity defects or to fill defects in the iliac crest, for example, resulting from the removal of autograft. In embodiments, the devices may be implanted into bone defects, such as fracture voids, by impaction, to allow for natural bone remodeling or healing. In other embodiments, the device may be combined with bone marrow aspirate or blood prior to implantation. Such devices can be used to deliver mesenchymal stem cells to the implant site. These stem cells can differentiate at the implant site into bone-forming cells to promote healing and repair.

Methods to implant the devices are also described. Devices comprising a stem connected to a cap are preferably inserted into a burr hole such that the stem is lodged inside the burr hole, a flange on the cap is located in contact with the outer surface of the cranium, and filament elements present on the stem engage in the cancellous bone of the cranium. The devices preferably comprise ceramic, and are preferably located such that there is a higher concentration of ceramic in the device near the entrance to the burr hole than in the vicinity of the diploë of the cranium. In other embodiments, devices comprising a cylindrical hollow core with flange and a separate pin are implanted by inserting the cylindrical hollow core into the burr hole until the flange is flush with the outer surface of the cranium, and then inserting the pin into the cylindrical hollow core component.

In view of the foregoing, it is thus an object of the invention to provide medical devices to fill burr holes.

It is still another object of the invention to provide medical devices to fill bone holes, defects, or injuries.

It is still another object of the invention to provide medical devices that not only fill burr holes, but also prevent the formation of depressions in the cranium following trephination.

It is yet another object of the invention to provide medical devices to fill burr holes that prevent the formation of depressions in the cranium following trephination.

These and other objects, aspects, and advantages of the subject invention shall become apparent in view of the following description with reference to the accompanying figures.

BRIEF DESCRIPTION OF THE DRAWINGS

FIG. 1 is a diagram of a medical device (100) in accordance with an embodiment of the invention inserted in a burr hole (130) of the cranium (140), showing the stem (110), and cap (120) of the device, the inner cranium surface (160), the outer cranium surface (150), the diploe (170) and the meninges (180).

FIG. 2A is a front view of a medical device (200) in accordance with an embodiment of the invention for insertion into a burr hole, showing the stem (240) with filament elements or bristles (210), and cap (220) with flange (230).

FIG. 2B is a bottom side isometric view of the medical device (200) shown in FIG. 2A.

FIG. 2C is a top view of the medical device (200) shown in FIG. 2A illustrative of a triangular porous structure.

FIG. 3 is a figure illustrating a bottom view of a medical device (300) for filling a burr hole in accordance with an embodiment of the invention, prepared by 3D printing of P4HB with a filling density of 70%, a drop ratio of 1.3, average filament (310) diameters ($\phi_F$) of 285 $\mu$m, and average distances $D_{AVE}$ between the printed filaments of 200 $\mu$m.

FIG. 4 is a figure illustrating a bottom view of a medical device (400) for filling a burr hole in accordance with an embodiment of the invention, prepared by 3D printing of P4HB with a filling density of 65%, average distance between the printed filaments (410) of 250 $\mu$m, and a drop ratio of 1.3.

FIG. 5 is a figure illustrating a bottom view of a medical device (500) for filling a burr hole in accordance with an embodiment of the invention, prepared by 3D printing of P4HB with a filling density of 55%, average distance between filaments (510) of 350 $\mu$m, and a drop ratio of 1.3.

FIG. 6 is a figure illustrating a bottom view of a medical device (600) for filling a burr hole in accordance with an embodiment of the invention, prepared by 3D printing of P4HB with a filling density of 45%, average distance between filaments (610) of 500 $\mu$m, and a drop ratio of 1.3.

FIG. 7 is a figure illustrating a bottom view of a medical device (700) for filling a burr hole in accordance with an embodiment of the invention, prepared by 3D printing of P4HB with a filling density of 40%, average distance between filaments (710) of 575 $\mu$m, and a drop ratio of 1.3.

FIG. 8A is an isometric view of a device (800) for filling a burr hole in accordance with an embodiment of the invention showing a rivet push pin (810) inserted in a cylindrical hollow core (820) with a flange (830).

FIG. 8B is an exploded isometric view of the rivet push pin (810) and cylindrical hollow core (820) shown in FIG. 8A.

FIG. 8C is an exploded front view of the rivet push pin (810) and a cylindrical hollow core (820) shown in FIG. 8A.

FIG. 9A is an isometric view of a device (900) for filling a burr hole in accordance with an embodiment of the invention showing a threaded pin (910) inserted in a cylindrical hollow core (920).

FIG. 9B is a front view of the device (900) shown in FIG. 9A showing the threaded pin (910) inserted in the cylindrical hollow core (920).

FIG. 9C is a cross-section view of the device (900) shown in FIG. 9B taken along line 9C-9C.

FIG. 9D is a top view of the device (900) shown in FIG. 9A showing the flange (930) of the cylindrical hollow core (920), and the head of the threaded pin (940) with a hexagonal socket (960).

FIG. 9E is a bottom view of the device (900) shown in FIG. 9A showing the textured flange (950) of the cylindrical hollow core (920).-

FIG. 10A is a bottom isometric view of a medical device (1000) for filling a burr hole in accordance with an embodiment of the invention comprising a rectangular shaped open pore structure with average distance between filaments of 250 μm.

FIG. 10B is a bottom isometric view of another medical device (1020) for filling a burr hole in accordance with an embodiment of the invention comprising a parallelogram-shaped open pore structure, and average distance between filaments of 760 μm.

FIG. 11 is an enlarged portion of a medical device viewed from the top in accordance with an embodiment of the invention showing a triangular open pore structure with layers of crisscrossed filaments.

FIG. 12 is a side view of a cylindrical-shaped stem (1040) of a medical device for filling a burr hole in accordance with an embodiment of the invention, prepared by repeating each filament layer before changing the print angle of the filament layer, serving to create increased lateral porosity (L).

DETAILED DESCRIPTION OF THE INVENTION

Before the present invention is described in detail, it is to be understood that this invention is not limited to particular variations set forth herein as various changes or modifications may be made to the invention within the scope of the appended claims.

Methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

Reference to a singular item, includes the possibility that there are plural of the same items present. More specifically, as used herein and in the appended claims, the singular forms "a," "an," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Last, it is to be appreciated that unless defined otherwise, all technical and scientific terms used herein have the same meaning as commonly understood by one of ordinary skill in the art to which this invention belongs.

In embodiments of the invention, an implantable medical device closes a burr hole following a trephination procedure, reduces or prevents the formation of a depression on the outer surface of the cranium, provides an improved cosmetic result, reduces or eliminates palpability at the burr hole site, and reduces or eliminates functional handicaps encountered by the patient during hair dressing or combing. The medical device may be sized for use in different sizes of burr holes. The medical device may also be used in the repair of other bone defects. The medical devices are preferably resorbable, porous, and allow tissue in-growth.

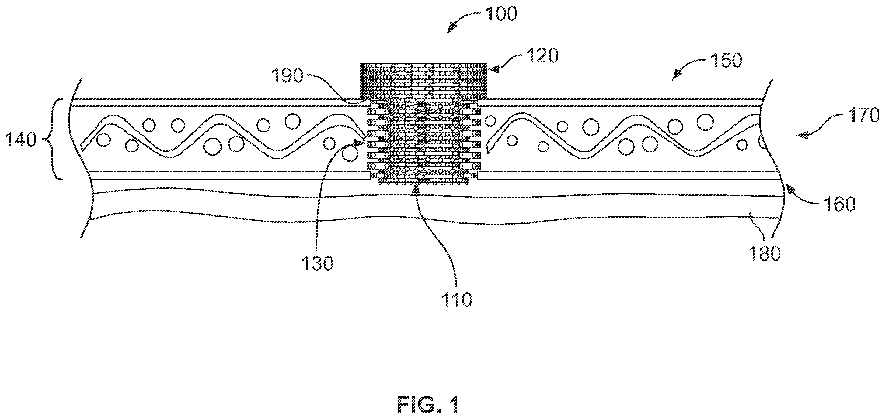

The device has a stem component connected to a cap component. The device is utilized by inserting the stem component into the burr hole until a flange of the cap component abuts the outer surface of the cranium. The device is preferably sized to prevent it from being dislodged from the burr hole. The device has filament elements emanating from the circumference of the stem that engage the cancellous tissue surrounding the burr hole when the device is inserted in the burr hole. The filament elements lock the device in place. In embodiments, the device expands after implantation to prevent its dislodgement from the burr hole. In other embodiments, the stem is glued in place. Preferably, the flange of the cap component prevents the stem component from protruding into the space between the inner surface of the cranium and the meninges. After implantation, tissue ingrowth into the device begins, and bone is regenerated in the burr hole. Over time, the device degrades, and is replaced with autologous bone. Figure 1 is a diagram showing the placement of a device (100) in a burr hole (130) with the stem (110) of the burr hole located through the diploë (170) of the cranium and the cap of the device located on the outer surface of the cranium. The flange (190) of the cap (120) extends beyond the circumference of the burr hole to ensure that the device cannot be extended beyond the inner surface (160) of the cranium and damage the underlying meninges (180) or be dislodged from the burr hole.

In embodiments, the device has a cylindrical hollow core component with an attached flange and a separate pin component. The device is utilized by implanting the cylindrical hollow core component in a burr hole, and inserting the pin component into the cylindrical hollow core component. Insertion of the pin component into the cylindrical hollow core component expands the cylindrical hollow core component, and causes the cylindrical hollow core component to apply pressure to the side walls of the burr hole which secures the device in the burr hole. The pin may be a push pin rivet. The pin's length may shorten as its diameter increases. The flange of the cylindrical hollow core component prevents the cylindrical hollow core component from protruding into the space between the inner surface of the cranium and the meninges. After implantation, tissue ingrowth into the device begins, and bone is regenerated in the burr hole. Over time, the device completely degrades, and is replaced with autologous bone unless the pin component is made from a permanent material.

In other embodiments, the pin of the device is threaded and is screwed into the cylindrical hollow core. In other embodiments, the pin is tapered and pushed into the cylindrical hollow core causing the diameter of the cylindrical hollow core to expand. In embodiments, the cylindrical hollow core further comprises a flange to prevent the cylindrical hollow core from being pushed too far through the burr hole and into the space between the inner cranium surface and the meninges.

In embodiments, the implanted device has a higher concentration of ceramic, lower porosity, or both a higher concentration of ceramic and lower porosity, at the entry point to the burr hole than it has within the dipolë region of the burr hole (i.e. in the cancellous region of the cranium). After implantation, the higher concentration of ceramic and or lower porosity of the device helps to regenerate hard tissue at the entry point to the burr hole. The regenerated hard tissue reduces or eliminates palpability in the area of the burr hole, and reduces or eliminates the formation of depressions in the cranium at the entry point to the burr hole.

I. DEFINITIONS

"Absorbable" as generally used herein means the material is degraded in the body, and the degradation products are eliminated or excreted from the body. The terms "absorbable", "resorbable", "degradable", and "erodible", with or without the prefix "bio", can be used interchangeably herein, to describe materials broken down and gradually absorbed, excreted, or eliminated by the body.

"Bioactive agent" is used herein to refer to therapeutic, prophylactic or diagnostic agents, preferably agents that promote healing and the regeneration of host tissue, and also therapeutic agents that prevent, inhibit or eliminate infection. "Agent" includes a single such agent and is also intended to include a plurality.

"Biocompatible" as generally used herein means the biological response to the material or device being appropriate for the device's intended application *in vivo.* Any metabolites of these materials should also be biocompatible.

"Blend" as generally used herein means a physical combination of different polymers, as opposed to a copolymer formed of two or more different monomers.

"Burr hole" as generally used herein means a small hole made in the skull.

"Calvaria" as used herein means the skullcap.

"Copolymers of poly(butylene succinate)" as generally used herein means any polymer containing 1,4-butanediol units and succinic acid units with one or more different diols, diacid or hydroxycarboxylic acid units, including hydroxycarboxylic acid groups with one or more carboxylic acid or hydroxy acid groups. The copolymers may also comprise chain extenders, coupling agents, cross-linking agents or branching agents.

"Copolymers of poly-4-hydroxybutyrate" as generally used herein means any polymer containing 4-hydroxybutyrate with one or more different hydroxy acid units.

"Diploë" is the region in the cranium that is located between the two outer cortical layers of bone. It comprises cancellous bone.

"Drop ratio" as used herein means the ratio of the drop width to the drop height during 3D printing.

"Elongation to break" as used herein means the increase in length of a material that occurs when tension is applied to break the material. It is expressed as a percentage of the material's original length.

"Endotoxin units" as used herein are determined using the limulus amebocyte lysate (LAL) assay as further described by Gorbet et al. Biomaterials, 26:6811-6817 (2005).

"Filling density" as used herein is the ratio of volume covered by the 3D printed material divided by the overall volume of the 3D printed object expressed as percent infill.

"Discharge number" as used herein is defining the droplet output.

"Macro-porous" materials or structures as used herein have average pore size diameters of at least 25 microns, more preferably at least 50 microns, and even more preferably at least 75 microns.

"Molecular weight" as used herein, unless otherwise specified, refers to the weight average molecular weight (Mw), not the number average molecular weight (Mn), and is measured by GPC relative to polystyrene.

"Oriented" as generally used herein refers to molecular alignment of polymer chains in a material. A polymer that has been stretched becomes partly oriented and then highly oriented, and the tensile strength increases with increasing orientation. For example, an unoriented polymeric fiber may be stretched to orient the fiber which results in a polymeric fiber with higher tensile strength.

"Poly-4-hydroxybutyrate" as generally used herein means a homopolymer containing 4-hydroxybutyrate units. It can be referred to herein as Tepha's P4HB™ polymer or TephaFLEX® biomaterial (manufactured by Tepha, Inc., Lexington, MA).

"Poly(butylene succinate)" as generally used herein means a polymer containing 1,4-butanediol units and succinic acid units.

"Trephination" as used herein means the process of creating a burr hole in the skull.

II. MATERIALS FOR PREPARING DEVICES FOR RECONSTRUCTION OF THE CRANIUM FROM HERE

In accordance with embodiments of the invention described herein, implantable medical devices, namely devices to fill burr holes, reduce or prevent the formation of depressions in the cranium after trephination, and reduce or eliminate physical handicaps during hairdressing or combing are disclosed. The devices are porous, and are inserted into burr holes in order to repair the burr holes without the formation of depressions on the outer surface of the cranium. Sealing the burr holes protects the underlying tissues and brain from infection or other potential injuries. The devices preferably comprise resorbable materials unless a subsequent procedure is planned, and more preferably comprise resorbable polymers that provide a scaffold structure for tissue in-growth, and gradually degrade as they are replaced with autologous tissue.

With reference again to FIG. 1, an embodiment of a device (100) for insertion in a burr hole (130) of the cranium (140) in accordance with the subject of the invention is shown. As described further herein, the device (100) is porous, and has a stem component (110) for placement in a burr hole (130), and a connected cap component (120) with a flange (190) that abuts the outer surface (150) of the cranium when the device (100) is inserted into a burr hole. The device further comprises filament elements emanating from the circumference of the stem to allow the stem to be anchored in the burr hole. In embodiments, the stem component (110) of the device (100) has a diameter sized to fit a burr hole. In embodiments, the filament elements emanating from the stem (110) are sized to penetrate the cancellous bone of the cranium when the device is implanted. The device remodels in vivo to seal the burr hole without the formation of a depression in the outer surface of the cranium. In embodiments, the cap has a convex shape to prevent the formation of depressions at the burr hole site. The device has a composition and porosity that prevents or reduces the formation of a depression at the entry point to the burr hole. The devices reduce or eliminate palpability due to the formation of a depression in the cranium at the site of the burr hole, or due to the formation of soft tissue (instead of hard tissue) at this site.

In other embodiments, the device has a cylindrical hollow core component with a flange that is inserted into a burr hole, and a separate pin that is inserted into the cylindrical hollow core component to secure the device in the burr hole. The device remodels in vivo to seal the burr hole without the formation of a depression in the outer surface of the cranium. In embodiments, the pin has a convex head to prevent or reduce the formation of a depression at the entry point to the burr hole. The device has a composition and porosity that prevents or reduces the formation of a depression at the entry point to the burr hole. The devices reduce or eliminate palpability due to the formation of a depression in the cranium at the site of the burr hole, or due to the formation of soft tissue (instead of hard tissue) at this site. In embodiments, the pin may be made from a permanent polymer to allow its removal for a subsequent procedure using the same burr hole, and eliminating the need to drill a new burr hole.

The medical devices are preferably made of absorbable polymers if subsequent procedures are not contemplated. Additionally, the devices may be molded or made from a single component, such as a filament. The filament may be unoriented, partially or fully oriented, and the filament may be 3D printed. The medical devices may optionally comprise bioactive agents, as well as cells, including stem cells. The medical devices so formed preferably have a pyrogen level of less than 20 endotoxin units per device, and can be sterilized.

A. Materials

The medical devices may comprise permanent and or degradable materials, and more preferably are made completely from degradable materials unless a subsequent procedure is expected. In a preferred embodiment, the devices for sealing burr holes are made from one or more absorbable polymers, preferably absorbable thermoplastic polymers and copolymers. The implantable devices may, for example, be prepared from polymers including, but not limited to, polymers of glycolic acid, lactic acid, 1,4-dioxanone, trimethylene carbonate, 3-hydroxybutyric acid, 4-hydroxybutyric acid, ε-caprolactone, 1,4-butanediol, and succinic acid, including polyglycolic acid, polylactic acid, polydioxanone, polycaprolactone, copolymers of glycolic and lactic acids, such as VICRYL® polymer, MAXON® and MONOCRYL® polymers, and including poly(lactide-co-caprolactones); poly(orthoesters); polyanhydrides; poly(phosphazenes); polyhydroxyalkanoates (PHA's); synthetically or biologically prepared polyesters; polycarbonates; tyrosine polycarbonates; polyamides (including synthetic and natural polyamides, polypeptides, and poly(amino acids)); polyesteramides; poly(alkylene alkylates); polyethers (such as polyethylene glycol, PEG, and polyethylene oxide, PEO); polyvinyl pyrrolidones or PVP; polyurethanes; polyetheresters; polyacetals; polycyanoacrylates; poly(oxyethylene)/poly(oxypropylene) copolymers; polyacetals, polyketals; polyphosphates; (phosphorous-containing) polymers; polyphosphoesters; polyalkylene oxalates; polyalkylene succinates; poly(maleic acids); silk (including recombinant silks and silk derivatives and analogs); chitin; chitosan; modified chitosan; biocompatible polysaccharides; hydrophilic or water soluble polymers, such as polyethylene glycol, (PEG) or polyvinyl pyrrolidone (PVP), with blocks of other biocompatible or biodegradable polymers, for example, poly(lactide), poly(lactide-co-glycolide), or polycaprolactone and copolymers thereof, including random copolymers and block copolymers thereof. Preferably the absorbable polymer or copolymer will be substantially or completely resorbed two years after implantation.

Blends of polymers, preferably absorbable polymers, can also be used to prepare the medical devices. Particularly preferred blends of absorbable polymers include, but are not limited to, polymers of glycolic acid, lactic acid, 1,4-dioxanone, trimethylene carbonate, 3-hydroxybutyric acid, 4-hydroxybutyric acid, ε-caprolactone, 1,4-butanediol, succinic acid or copolymers thereof.

In a particularly preferred embodiment, the medical devices comprise poly-4-hydroxybutyrate (Tepha's P4HB™ polymer, Lexington, MA) or a copolymer thereof, and may in one embodiment be made completely with P4HB or copolymer thereof. Copolymers include P4HB with another hydroxyacid, such as 3-hydroxybutyrate, and P4HB with glycolic acid or lactic acid monomer. P4HB is a strong, pliable thermoplastic polyester that is biocompatible and resorbable (Williams, et al. Poly-4-hydroxybutyrate (P4HB): a new generation of resorbable medical devices for tissue repair and regeneration, Biomed. Tech. 58(5):439-452 (2013)). Upon implantation, P4HB hydrolyzes to its monomer, and the monomer is metabolized via the Krebs cycle to carbon dioxide and water. In a preferred embodiment, the P4HB homopolymer and copolymers thereof have a weight average molecular weight, Mw, within the range of 50 kDa to 1,200 kDa (by GPC relative to polystyrene) and more preferably from 100 kDa to 600 kDa. A weight average molecular weight of the polymer of 50 kDa or higher is preferred for processing and mechanical properties.

In another preferred embodiment, the medical devices comprise a polymer comprising at least a diol and a diacid. In a particularly preferred embodiment, the polymer used to prepare the device is poly(butylene succinate) (PBS) wherein the diol is 1,4-butanediol and the diacid is succinic acid. The poly(butylene succinate) polymer may be a copolymer with other diols, other diacids or a combination thereof. For example, the polymer may be a poly(butylene succinate) copolymer that further comprises one or more of the following: 1,3-propanediol, 2,3-butanediol, ethylene glycol, 1,5-pentanediol, glutaric acid, adipic acid, terephthalic acid, malonic acid, methylsuccinic acid, dimethylsuccinic acid, and oxalic acid. Examples of preferred copolymers are: poly(butylene succinate-co-adipate), poly(butylene succinate-co-terephthalate), poly(butylene succinate-co-butylene methylsuccinate), poly(butylene succinate-co-butylene dimethylsuccinate), poly(butylene succinate-co-ethylene succinate) and poly(butylene succinate-co-propylene succinate). The poly(butylene succinate) polymer or copolymer may also further comprise one or more of the following: chain extender, coupling agent, cross-linking agent and branching agent. For example, poly(butylene succinate) or copolymer thereof may be branched, chain extended, or cross-linked by adding one or more of the following agents: malic acid, trimethylol propane, trimesic acid, citric acid, glycerol propoxylate, and tartaric acid. Particularly preferred agents for branching, chain extension, or crosslinking the poly(butylene succinate) polymer or copolymer thereof are hydroxycarboxylic acid units. Preferably the hydroxycarboxylic acid unit has two carboxylic groups and one hydroxyl group, two hydroxyl groups and one carboxyl group, three carboxyl groups and one hydroxyl group, or two hydroxyl groups and two carboxyl groups. In one preferred embodiment, the device comprises poly(butylene succinate) comprising malic acid as a branching, chain extending, or cross-linking agent. This polymer may be referred to as poly(butylene succinate) cross-linked or chain-extended with malic acid, succinic acid-1,4-butanediol-malic acid copolyester, or poly(1,4-butylene glycol-co-succinic acid), cross-linked or chain-extended with malic acid. It should be understood that references to malic acid and other cross-linking agents, coupling agents, branching agents and chain extenders include polymers prepared with these agents wherein the agent has undergone further reaction during processing. For example, the agent may undergo dehydration during polymerization. Thus, poly(butylene succinate)-malic acid copolymer refers to a copolymer prepared from succinic acid, 1,4-butanediol and malic acid. In another preferred embodiment, malic acid may be used as a branching, chain-extending or cross-linking agent to prepare a copolymer of poly(butylene succinate) with adipate, which may be referred to as poly[(butylene succinate)-co-adipate] cross-linked or chain-extended with malic acid. As used herein, "poly(butylene succinate) and copolymers" includes polymers and copolymers prepared with one or more of the following: chain extenders, coupling agents, cross-linking agents and branching agents. In a particularly preferred embodiment, the poly(butylene succinate) and copolymers thereof contain at least 70%, more preferably 80%, and even more preferably 90% by weight of succinic acid and 1,4-butanediol units. The polymers comprising diacid and diols, including poly(butylene succinate) and copolymers thereof and others described herein, preferably have a weight average molecular weight (Mw) of 10,000 Da to 400,000 Da, more preferably 50,000 Da to 300,000 Da and even more preferably 100,000 Da to 200,000 Da based on gel permeation chromatography (GPC) relative to polystyrene standards. In a particularly preferred embodiment, the polymers and copolymers have a weight average molecular weight of 50,000 Da to 300,000 Da, and more preferably 75,000 Da to 300,000 Da. In one preferred embodiment, the poly(butylene succinate) or copolymer thereof used to make the device, or a component of the device, has one or more, or all of the following properties: density of 1.23-1.26 g/cm$^3$, glass transition temperature of -31 °C to -35 °C, melting point of 113 °C to 117 °C, melt flow rate (MFR) at 190 °C/2.16 kgf of 2 to 10 g/10 min, and tensile strength of 30 to 60 MPa.

When the burr hole may be accessed in a subsequent procedure, the device may comprise a component made from a permanent material. For example, the device may comprise a permanent pin as disclosed further herein. Permanent materials that can be used to prepare components of the device, such as a pin, include: metals, alloys, ceramics and non-degradable polymers. Examples of suitable metals and alloys include stainless steel, tantalum, titanium, cobalt-chromium, iron, zirconium, manganese, and magnesium alloys, and Nitinol. Examples of suitable non-degradable polymers that can be used to prepare components of the device, such as a pin, include polymers and copolymers of ethylene and propylene, including ultra-high molecular weight polyethylene, ultra-high molecular weight polypropylene, nylon, polyesters such as polyethylene terephthalate), poly(tetrafluoroethylene), polyurethanes, poly(ether-urethanes), poly(methylmethacrylate), polyether ether ketone, polyolefins, and poly(ethylene oxide).

B. Additives

Certain additives may be incorporated into the devices, preferably in the absorbable polymer, copolymer or blends thereof that are used to make the device. These additives may be incorporated during a compounding process subsequent to fabrication of the device. For example, additives may be melt compounded with polymers, or compounded using a solution-based process.

In a preferred embodiment, the additives are biocompatible, and even more preferably the additives are both biocompatible and resorbable.

In one embodiment, the additives may be nucleating agents and/or plasticizers. These additives may be added in sufficient quantity to produce the desired result. In general, these additives may be added in amounts between 1% and 20% by weight. Nucleating agents may be incorporated to increase the rate of crystallization of the polymer, copolymer or blend. Such agents may be used, for example, to facilitate fabrication of the device, and to improve the mechanical properties of the device. Preferred nucleating agents include, but are not limited to, salts of organic acids such as calcium citrate, polymers or oligomers of PHA polymers and copolymers, high melting polymers such as PGA, talc, micronized mica, calcium carbonate, ammonium chloride, and aromatic amino acids such as tyrosine and phenylalanine.

Plasticizers that may be incorporated into the compositions for preparing the devices include, but are not limited to, di-n-butyl maleate, methyl laureate, dibutyl fumarate, di(2-ethylhexyl) (dioctyl) maleate, paraffin, dodecanol, olive oil, soybean oil, polytetramethylene glycols, methyl oleate, n-propyl oleate, tetrahydrofurfuryl oleate, epoxidized linseed oil, 2-ethyl hexyl epoxytallate, glycerol triacetate, methyl linoleate, dibutyl fumarate, methyl acetyl ricinoleate, acetyl tri(n-butyl) citrate, acetyl triethyl citrate, tri(n-butyl) citrate, triethyl citrate, bis(2-hydroxyethyl) dimerate, butyl ricinoleate, glyceryl tri-(acetyl ricinoleate), methyl ricinoleate, n-butyl acetyl rincinoleate, propylene glycol ricinoleate, diethyl succinate, diisobutyl adipate, dimethyl azelate, di(n-hexyl) azelate, tri-butyl phosphate, and mixtures thereof. Particularly preferred plasticizers are citrate esters.

C. Bioactive Agents

The medical devices can be loaded or coated with bioactive agents. Bioactive agents may be included in the devices for a variety of reasons. For example, bioactive agents may be included in order to improve tissue in-growth into the device, to improve tissue maturation, for example, bone formation, to provide for the delivery of an active agent, to improve wettability of the implant, to prevent infection, and to improve cell attachment. The bioactive agents may also be incorporated into different regions of the device in different concentrations. For example, to promote the formation of cortical or cancellous bone in the cranium.

In a preferred embodiment, the bioactive agents are ceramics. More preferably, the bioactive agents are bioceramics, and more preferably resorbable bioceramics. The ceramics help to promote bone formation in the burr hole and at the entry point to the burr hole. The ceramics are preferably osteoinductive ceramics. Osteoinductive ceramics help in the process of bone formation. In embodiments, the ceramics include calcium phosphates, calcium orthophosphates, hydroxyapatites, $\alpha$-tricalcium phosphate, $\beta$-tricalcium phosphate ($\beta$-TCP), sintered hydroxyapatite, precipitated hydroxyapatite, monocalcium phosphate monohydrate, dicalcium phosphate dihydrate, dicalcium phosphate anhydrous, amorphous calcium phosphate, tetracalcium phosphate, and octacalcium phosphate. A particularly preferred ceramic is $\beta$-TCP. The amount of ceramic in the device may be 0.1 to 80 wt. %, or more preferably 5 to 50 wt. %.

The devices may contain cellular adhesion factors, including cell adhesion polypeptides. As used herein, the term "cell adhesion polypeptides" refers to compounds having at least two amino acids per molecule that are capable of binding cells via cell surface molecules. The cell adhesion polypeptides include any of the proteins of the extracellular matrix which are known to play a role in cell adhesion, including fibronectin, vitronectin, laminin, elastin, fibrinogen, collagen types I, II, and V, as well as synthetic peptides with similar cell adhesion properties. The cell adhesion polypeptides also include peptides derived from any of the aforementioned proteins, including fragments or sequences containing the binding domains.

The devices can incorporate wetting agents designed to improve the wettability of the surfaces of the device to allow fluids to be easily adsorbed onto the device surfaces and into porous devices, and to promote cell attachment and or modify the water contact angle of the device surface. Examples of wetting agents include polymers of ethylene oxide and propylene oxide, such as polyethylene oxide, polypropylene oxide, or copolymers of these, such as PLURONICS®. Other suitable wetting agents include surfactants or emulsifiers.

The devices can contain gels, hydrogels or living hydrogel hybrids to further improve wetting properties and to promote cellular growth throughout the thickness or diameter of the device. Hydrogel hybrids consist of living cells encapsulated in a biocompatible hydrogel like gelatin, silk gels, and hyaluronic acid (HA) gels.

The devices can contain active agents designed to stimulate cell in-growth, including growth factors, cellular differentiating factors, cellular recruiting factors, cell receptors, cell-binding factors, cell signaling molecules, such as cytokines, and molecules to promote cell migration, cell division, cell proliferation and extracellular matrix deposition. Such active agents include fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleukin-1-B (IL-1B), interleukin-8 (IL-8), and nerve growth factor (NGF), and combinations thereof.

Other bioactive agents that can be incorporated in the devices include antimicrobial agents, in particular antibiotics, disinfectants, oncological agents, anti-scarring agents, antiinflammatory agents, anesthetics, small molecule drugs, anti-angiogenic factors and pro-angiogenic factors, immunomodulatory agents, and blood clotting agents. The bioactive agents may be proteins such as collagen and antibodies, peptides, polysaccharides such as chitosan, alginate, hyaluronic acid and derivatives thereof, nucleic acid molecules, small molecular weight compounds such as steroids, inorganic materials such as hydroxyapatite, or complex mixtures such as platelet rich plasma. Suitable antimicrobial agents include: bacitracin, biguanide, triclosan, gentamicin, minocycline, rifampin, vancomycin, cephalosporins, copper, zinc, silver, and gold. Nucleic acid molecules may include DNA, RNA, siRNA, miRNA, antisense or aptamers.

In yet another preferred embodiment, the devices may incorporate systems for the controlled release of therapeutic or prophylactic agents.

D. Filaments

The devices comprise filaments. The filaments are preferably made from degradable thermoplastic polymers, and even more preferably from degradable thermoplastic polyesters. The filaments are preferably made from the degradable materials listed in section II.A above. In a preferred embodiment, the filaments are made from P4HB or copolymer thereof. In another preferred embodiment, the filaments are made from poly(butylene succinate) or copolymer thereof. The filaments maybe 3D printed, monofilament fibers, multifilament fibers, or combinations thereof. The filaments may be a yarn that is twisted, not twisted, or substantially parallel strands. The filaments may be unoriented, partially oriented, highly oriented or combinations thereof. Preferably, the filaments are unoriented. The filaments may have diameters ranging from 0.05 to 0.8 mm, more preferably 0.1 to 0.4 mm, and even more preferably 0.15 to 0.3 mm. The weight average molecular weights of the filament polymers may be 10 kDa to 1,200 kDa, but are more preferably 50 kDa to 600 kDa. Preferably the filaments have a tensile modulus of 10 to 1,000 MPa, and more preferably 30 to 300 MPa, and even more preferably 30 to 60 MPa. The filaments may have short degradation profiles, prolonged degradation profiles, or combinations thereof. In one embodiment, a short degradation profile is 1 to 12 weeks, and a prolonged degradation profile is from 12 weeks to 5 years, more preferably 4 months to 2 years. The filaments of the device may have different degradation rates *in vivo.* Some filaments may degrade quickly while other filaments may degrade slowly. In another embodiment, the filaments comprise an additive or bioactive agent. In a preferred embodiment, the filaments comprise a ceramic, more preferably a resorbable bioceramic, and even more preferably, the filaments comprise β-TCP.

The filaments can be produced by any suitable method such as 3D printing, melt extrusion, and solvent spinning but 3D printing is preferred. In a particularly preferred embodiment, the filaments are produced by melt extrusion deposition.

In embodiments, the filament are produced by melt extrusion deposition of P4HB or copolymer thereof, or melt extrusion deposition of poly(butylene succinate) or copolymer thereof. More preferably, the filaments are produced by melt extrusion deposition of blends of P4HB and β-TCP or blends of poly(butylene succinate) or copolymer thereof with β-TCP.

Devices that can fill burr holes can be prepared from the filaments described above. Such devices can be produced from slow and fast degrading filaments, degradable filaments of different molecular weights, filaments that are unoriented, partially oriented and fully oriented, filaments with different elongation to break values, tensile strengths and tensile modulus values, or combinations thereof.

E. Foams and Porous Constructs

The devices may comprise foams or other porous constructs. These constructs are preferably made from degradable thermoplastic polymers, and even more preferably from degradable thermoplastic polyesters. The constructs are preferably made from the degradable materials listed in Section II.A above. The constructs can be made by any suitable method, including melt foaming and solution foaming, particulate leaching and phase separation techniques. In a preferred embodiment, the porous constructs are made by 3D printing. In a preferred embodiment, the constructs are made from P4HB or copolymer thereof or poly(butylene succinate) or copolymer thereof. The constructs may optionally be cross-linked. Preferably the polymers of the constructs have weight average molecular weights of 10 kDa to 1,200 kDa, but more preferably 50 kDa to 600 kDa. The constructs may have open cell or closed cell structures. In one embodiment, the constructs have an open cell content of at least 10%, preferably at least 25%, and more preferably at least 50%. The cell sizes may be up to 5 mm. The densities of the constructs are preferably less than 1 g/cm$^3$, and more preferably less than 0.75 g/cm$^3$. The foams may have short degradation profiles of 1 to 12 weeks, or prolonged degradation profiles of 12 weeks to 5 years, or 12 weeks to 2 years. The constructs may comprise additives or bioactive agents. Preferably, the constructs further comprise ceramics, and more preferably resorbable bioceramics. Even more preferably, the constructs comprise P4HB and β-TCP, or poly(butylene succinate) or copolymer thereof and β-TCP.

Devices to fill burr holes can be prepared from the constructs described above. Such devices can be produced from foams and porous constructs with open or closed cell structures, varying cell sizes and densities, different molecular weights, and different degradation profiles.

III. METHODS OF MANUFACTURING DEVICES FOR RECONSTRUCTION OF BONE DEFECTS INCLUDING BURR HOLES

A variety of methods can be used to manufacture the medical devices for repairing bone defects, and specifically reconstructing burr holes, and several different examples are described herein. The devices reduce or eliminate the formation of depressions on the outer surface of the cranium at the entry points to burr holes. The devices are preferably replaced by hard tissue instead of soft tissue at the entry points to the burr holes which decreases or eliminates palpability at the burr hole site. In embodiments, the devices repair burr holes without the formation of depressions in the cranium at the burr hole entry point that are greater than 1 mm relative to the outer surface of the cranium. Prevention of depressions are important in providing an improved cosmetic outcome as well as preventing handicaps for hairdressing and combing.

The devices are implanted in burr holes in order to close the burr hole. The devices prevent the tissues beneath the burr hole and the brain from being infected or from potential injury. The devices minimize the formation of depressions in the cranium or eliminate the formation of depressions. The devices provide an improved cosmetic result, and reduce or eliminate functional handicaps which include combing and hairdressing. The devices allow tissue in-growth. The devices may provide a scaffold structure to allow tissue in-growth. The devices are preferably partially or completely resorbable, but in embodiments may comprise a permanent component which can be removed to facilitate a second neurosurgical procedure using the same burr hole. The devices preferably degrade in less than 5 years, more preferably in less than 2 years, and even more preferably in less than 1 year. The devices preferably comprise resorbable polymers. The devices are preferably completely resorbed after implantation and replaced with autologous bone, preferably calvarial bone. Resorption of the devices prevents any interference with imaging techniques such as CT and magnetic resonance imaging. The devices create hard tissue at the entry point to the burr hole which is not palpable. The devices allow an early and stable integration in the cranium, and are replaced over time as they are resorbed and replaced with new hard tissue.

The devices have three-dimensional shapes, and can be unitary or comprised of two or more components. For example, the devices may be unitary with a stem connected to a cap. Or the devices may comprise two components such as a cylindrical hollow core and a pin, wherein the pin can be inserted in the hollow core. The cylindrical hollow core and pin may both be made from a resorbable material. Alternatively, the pin can be made from a permanent material so that it can be removed for a subsequent procedure without a surgeon needing to drill another burr hole in the patient's cranium.

The devices are preferably dimensioned so that they can be inserted and secured in a burr hole. Preferably, the devices have a diameter that is similar in size to the diameter of the burr hole. In embodiments, the difference in the external diameter of the stem of the device or the cylindrical hole core and the diameter of the burr hole is ±2 mm, and more preferably ±1 mm. The devices are preferably dimensioned so that after implantation there is a tight fit of the device in the burr hole. In embodiments, the devices may be glued in place, for example, with fibrin glue. The devices preferably have a shape that prevents the device from protruding into the space between the inner surface of the cranium and the meninges. The devices preferably have flanges to prevent them from emerging from the burr hole in close proximity to the meninges.

In a particularly preferred embodiment, the devices comprise poly-4-hydroxybutyrate or copolymer thereof or poly(butylene succinate) or copolymer thereof, even more preferably in the form of porous constructs.

The devices may comprise the additives listed in Section II.B and the bioactive agents listed in Section II.C. The devices may be coated with one or more of the following: a bioactive agent, antibiotic, and an antimicrobial.

The manufactured devices preferably have an endotoxin content of less than 20 endotoxin units making them suitable for implantation in a patient. The manufactured devices are preferably sterile. In embodiments, the devices are sterilized by gamma-irradiation, electron beam irradiation or with ethylene oxide gas, preferably cold ethylene oxide gas.

A. Examples of devices for reconstruction of burr holes

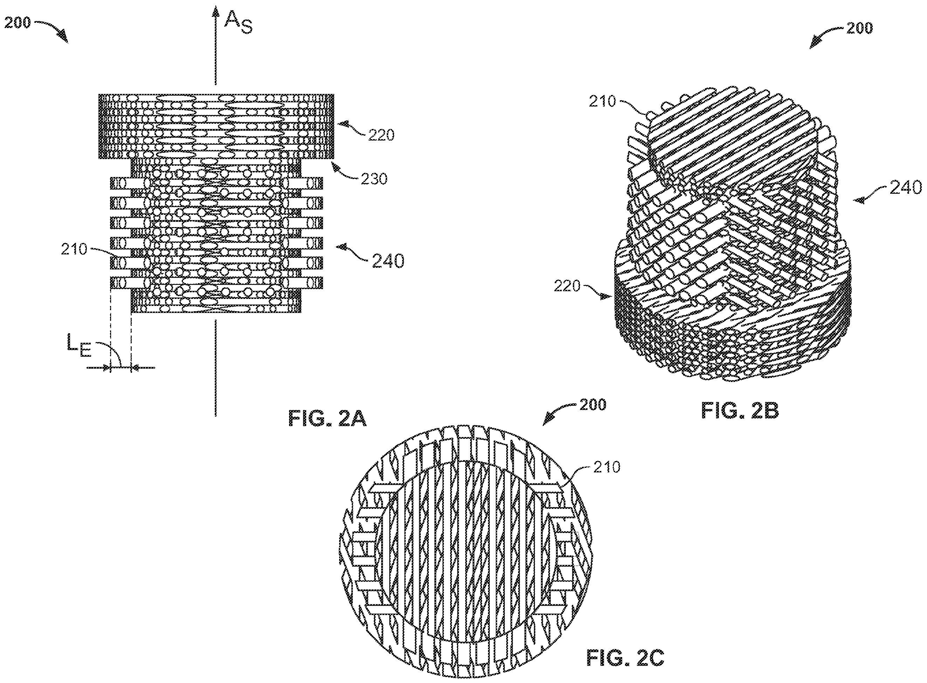

The devices are designed to repair burr holes using a stem component connected to a cap component with a flange section. A diagram showing a device (200) with a stem component (240), a cap component (220) and a flange (230) is shown in FIGS. 2A, 2B and 2C. The device comprises a stem component (240) suitable for placement in a burr hole, and a cap component (220) suitable for placement at the entry point to the burr hole. The flange (230) abuts the outer surface of the cranium when the device is inserted in a burr hole, and is sized to prevent the device from being pushed too far into the burr hole. In particular, the flange prevents the stem of the device from extending into the space between the inner cranium surface and the meninges.

The device (200) shown in FIGS. 2A-2C has filament elements (210) that are designed to secure the device in place once it is inserted in the burr hole. The filament elements are designed to be flexible so that they bend during insertion of the device in the burr hole, but then push into the cancellous bone of the cranium to hold the device in place. The filament elements (240) have a brush-like or bristle-like shape and stiffness.

The orientation and arrangement of the filament elements may vary. In embodiments, and as shown in FIG. 2A, the array of bristles extends in a traverse direction (about 90 degrees) from the stem axis ($A_S$).

In other embodiments, an array of bristles extends at an angle from the stem axis. The angle of the bristles from the stem axis may range from 0 to 90 degrees, or 30 to 60 degrees, and in some embodiments 40-50 degrees. Arranging the bristles at an acute angle from the stem axis does not impede insertion of the stem but, in contrast, prohibits retraction of the stem from the burr hole. Particularly, as the stem is retracted from the burr hole, the bristles are biased to become further lodged in the bone mass. In an embodiment, the device (200) is self-locking.

In embodiments, the cap component (220) has a convex shape. The convex shape is designed to minimize the formation of a depression at the entry point to the burr hole. The diameter of the stem (240) is sized to fit in the burr hole. Preferably the diameter of the stem is within ±2 mm, more preferably ±1 mm, of the diameter of the burr hole. The diameter of the flange (230) is larger than the diameter of the burr hole.

The filament elements (240) preferably have a length that is less than half the length of the stem (240) of the device. The length ($L_E$) of the element is measured from the circumference of the stem (240) to the outermost tip of the filament element (210). In embodiments, the length ($L_E$) of the element is 0.1 to 5 mm, and more preferably 1-2 mm. In embodiments, the diameter of the element is 0.15 to 0.8 mm. In embodiments, the device is solid, but in more preferred embodiments, the device (200) is porous and even more preferably comprises a resorbable polymer that over time is replaced with in-grown hard tissue. In embodiments, the device (200) comprises filaments. The filaments are preferably 3D printed. The entire device including the filament elements may be 3D printed.

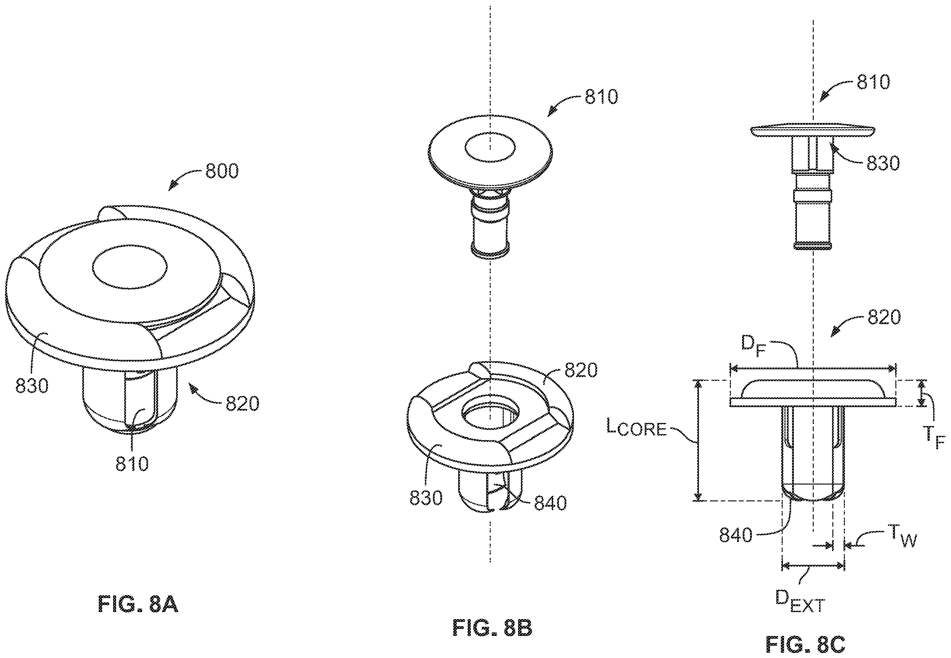

In another example, devices are designed to repair burr holes using a cylindrical hollow core component and a separate pin that is inserted in the hollow core. A diagram showing a device (800) with a pin (810) inserted in a cylindrical hollow core component (820) is shown in FIG. 8A. The cylindrical hollow core component (820) preferably comprises a flange (830). The device is implanted in a burr hole by pushing the cylindrical hollow core component into a burr hole until the flange of the cylindrical hollow core component is pressed against the outer surface of the cranium surrounding the burr hole. The pin (810) shown in FIG. 8B is then pushed into the cylindrical hollow core component (820) shown in FIG. 8B in order to anchor the device (800) in the burr hole, and close the burr hole. The pin (810) may be a rivet push pin (see FIG. 8C) designed to apply pressure on the cylindrical hollow core when inserted in the core, and force the core (820) into a secure contact with the inside of the burr hole. The pin (810) may be designed so that at least part of its diameter is larger than the diameter of the cylindrical hollow core. The pin (810) may also be designed so that its diameter increases as the pin is inserted inside the cylindrical hollow core. In an embodiment, the cylindrical hollow core has a pin stop (840) shown in FIG. 8B, which prevents the pin from advancing completely through the core. The pin stop (840) may be used in conjunction with a rivet push pin (810) of the type shown in FIG. 8B, which is designed to be compressed inside the cylindrical hollow core (820), latch in place as shown in the assembled device (800), and exert a transverse force on the cylindrical hollow core to secure the device in place in a burr hole. In an embodiment, the head of the pin (810) may have a convex shape. The convex shape is designed to reduce the formation of a depression at the entry point to the burr hole.

The dimensions of the device may vary except where limited by any appended claims. With reference to FIG. 8C, in embodiments of the invention, the external diameter ($D_{EXT}$) of the cylindrical hollow core (820) may range from 6 to 25 mm ±2 mm or 8 to 15 mm ±1 mm; the flange diameter ($D_F$) preferably extends at least 2 mm beyond the circumference of the external diameter of the cylindrical hollow core; the flange thickness ($T_F$) is preferably less than 5 mm; the length of the cylindrical hollow core ($L_{CORE}$) is preferably less than 11 mm; and the thickness of the wall ($T_W$) of the cylindrical hollow core, excluding the flange, can be about 3 mm.

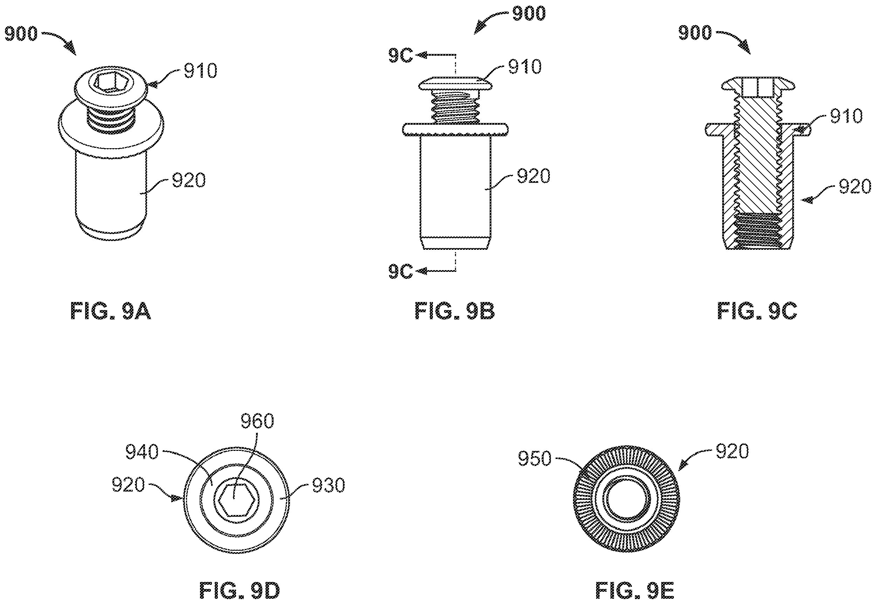

In a further preferred example, a cylindrical hollow core component may be used with a threaded pin to repair and close a burr hole. A diagram showing a device (900) with a threaded pin (910) partially inserted into a cylindrical hollow core component (920) is shown in FIGS. 9A and 9B. The cylindrical hollow core component (920) comprises a flange (930) as shown in FIG. 9D that correctly positions the device in a burr hole and prevents the device from protruding into the space between the inner cranium surface and the meninges. Once the cylindrical hollow core component is inserted in a burr hole with the flange (930) abutting the outer cranium surface, the threaded pin (910) is screwed into the cylindrical hollow core. A cross-section of the device (section A-A) showing a threaded pin (910) screwed into a cylindrical hollow core (920) is shown in FIG. 9C. The threaded pin (910) has a hexagonal socket (960) on its head (940), as shown in FIG. 9D, to allow rotation of the pin. The underside of the flange is preferably textured (950) as shown in FIG. 9E to prevent rotation of the cylindrical hollow core. The textured surface abuts the outer cranium surface and prevents rotation of the cylindrical hole core (920). Preferably, the threaded pin (910) is tapered so that once tightened inside the cylindrical hollow core (920) it applies pressure to the cylindrical hollow core so that the core is secured against the side wall of the burr hole. In an embodiment, the head of the pin (940) may have a convex shape. The convex shape is designed to reduce the formation of a depression at the entry point to the burr hole.

The pins (810) and (910) are designed to be removable for subsequent procedures. A removable pin allows the surgeon later access to the burr hole without needing to drill a new burr hole in the cranium. Removable pins (810) and (910) may be formed from permanent materials, including permanent polymers, ceramics and metals, including those listed in Section II. A.

B. Dimensions of devices for reconstruction of burr holes

Burr holes are typically drilled with diameters of 6 to 25 mm, more usually 8 to 19 mm although smaller and large holes can be made in the cranium. The devices disclosed herein are designed with stem component diameters (e.g. outer diameter of (240)) or cylindrical hollow core outer diameters (e.g. outer diameters of (820) and (920)) that are within ±2 mm, or more preferably ±1 mm, of the diameter of the burr hole. This design allows a tight fit of the device in the burr hole. In an embodiment, the diameter of the stem of the device (200) or the outer diameter of the cylindrical hollow core of the device, e.g. (820) or (920) is 6 to 25 mm ±2 mm, and more preferably 8 to 19 mm ± 1 mm.

In order to secure the device in the burr hole, the diameter of the pin (910) that is inserted in the cylindrical hollow core is preferably 0.5-2 mm, more preferably 0.5-1 mm, larger than the inner diameter of the cylindrical hollow core (920). For example, if the inner diameter of the cylindrical hollow core (920) is 6 mm, the pin (910) preferably has a diameter of 6.5 mm or 7 mm. The larger diameter of the pin compresses the cylindrical hollow core against the cancellous bone of the cranium, and secures the device at the implant site. In an embodiment, the desired diameter of the pin (910) is $d_{pin}$, and $d_{pin}$ may be calculated using the equation:

$$d_{pin} + 2 \times d_{thickness} = d_{hole} + 0.5 \text{ to } 2 \text{ mm}$$

wherein the diameter of the burr hole is $d_{hole}$, and the thickness of the wall of the cylindrical hollow core (920) is $d_{thickness}$.

The rivet push pin (810) is designed so that it has a first outer diameter that allows it to be inserted into the cylindrical hollow core, and a second expanded outer diameter that secures the device (800) in the burr hole. Preferably, the second expanded outer diameter is 0.5-2 mm, more preferably 0.5-1 mm, larger than the inner diameter of the cylindrical hollow core (820). The expansion of the rivet push pin (810) inside the cylindrical hollow core (820) compresses the core against the cancellous bone of the cranium securing the device (800) at the implant site.

Insertion of the pin enlarges the hollow core, causing an interference fit between the medical device and the bone hole, firmly securing the medical device in the hole.

The size of the device's flange (e.g. (230), (830) or (930)) must be sufficient to prevent the device passing through the burr hole and into the space between the inner cranium surface and the meninges. Preferably, the flange extends at least 2 mm, more preferably at least 3 mm beyond the circumference of the burr hole on the outer surface of the cranium, but less than 20 mm, more preferably less than 10 mm. For example, if the outer diameter of the stem component (e.g. 240) or cylindrical hollow core component (e.g. 820 or 920) is 14 mm, the diameter of the flange is preferably at least 18 mm, and more preferably at least 20 mm. Preferred diameters of the flange are 10 mm to 29 mm, but can be smaller or larger depending upon the size of the burr hole and the amount of overlap of the flange with the cranium desired. Preferably, the diameters of the flange are 12 mm to 19 mm.

The average thickness of the cranium for a male is 6.5 mm, and 7.1 mm for a woman. The devices disclosed herein are designed so that the burr hole is at least partly filled with the device, and may be completely filled by the device. However, the devices are designed so that the stem component (e.g., 240) of the devices (e.g. 200), or cylindrical hollow core component of the devices (e.g. 820 and 920), are not longer than the thicknesses of the cranium. This prevents the device from protruding into the space between the inner cranium surface (160) and the meninges (180) where it could potentially damage tissues. In an embodiment, the length of the stem of the device (e.g. 200) or cylindrical hollow core component of the device (e.g. 820 or 920) is less than 6 mm, more preferably less than 5 mm, and more preferably less than 4 mm.

It is preferred that the profile of the device protruding from the cranium on the outer cranium side (150) is relatively small after insertion of the device in the burr hole. Preferably, the cap component (220), for example, should not protrude above the surface of the cranium by more than 5 mm, more preferably by more than 3 mm, and even more preferably by more than 1 mm. Similarly, the profile of devices comprising a pin component and a cylindrical hollow core component, such as (800) and (900), should not, after implantation of the device in a burr hole, protrude above the surface of the cranium by more than 5 mm, more preferably by more than 3 mm, and even more preferably by more than 1 mm. In embodiments, it is preferable that the cap component (220) and the head of the pin components (810) and (910) have a convex shape. In an embodiment, the length of the device that is inserted into the burr hole measured in the longitudinal direction is 2 to 7 mm, more preferably 3 to 5 mm, and the length of the device measured in the longitudinal direction that is not inserted into the burr hole is 0.5 to 5 mm, and more preferably 1 to 2 mm.

C. Porosity of Devices

The devices for closing burr holes, including those shown in FIGS. 2-9 are preferably porous. The devices may be macro-porous. Preferably, the devices have a porosity of 50-70%. In embodiments, the devices are 3D printed and have filling densities of 0.4 to 0.7. In embodiments, the devices comprise P4HB or PBS, or copolymers thereof, and have densities of 0.2 to 0.6 g/cm$^3$. In an embodiment, the devices have an average pore size diameter, pore size dimension, or distance between filaments of 0.01 mm to 2 mm, more preferably 0.05 mm to 1 mm, and even more preferably 0.075 mm to 0.5 mm. The pore sizes of the device may be different in different regions of the device. The region of the device that is located within the burr hole may have larger pores or pore dimensions than the region of the device that is located at the entry point to the burr hole. A device (200) for insertion into a burr hole comprising a stem component (240) and a cap component (220) with a flange (230) which is designed to abut the outer surface of the cranium may have larger pores or larger pore dimensions in the stem component (240) than in the cap component (220) or flange component (230). For example, the average pore dimensions in the stem component (240) may be 0.05 to 2 mm, and 0.01 to 0.5 mm in the cap component (220) or flange component (230), wherein the average pore dimensions in the stem component are larger than the average pore dimensions in the flange component.

Devices comprising a cylindrical hollow core and pin, such as those shown in FIGS. 8 and 9, may be porous. A device (800) or (900) for insertion into a burr hole comprising a pin component (810) or (910) and a cylindrical hollow core component (820) or (920) may also have larger average pore dimensions or larger average pore sizes in the stem regions that are located inside the burr hole than in the flange regions (830) and (930). The cylindrical hollow core of this device design, such as (820) and (920) may be porous, and may have average pore size dimensions of 0.05 to 2 mm, and more preferably 0.075 to 1 mm. The pin of this device design, such as (810) and (910), may be solid, but can also be porous having average pore size dimensions of 0.075 mm to 1 mm. The pore size of the flange region of the cylindrical hollow core, such as (830) and (930), may be porous, and may have average pore size dimensions of 0.01 to 1 mm or 0.025 to 0.5 mm.

D. Devices comprising ceramics

The devices may comprise ceramics in amounts from 0.1 wt. % to 80 wt. %, more preferably 5 wt. % to 60 wt. %, and even more preferably 10 wt. % to 40 wt. %. Preferably the ceramic is blended with a resorbable polymer, and more preferably with P4HB or copolymer thereof or with poly(butylene succinate) or copolymer thereof. The amount of ceramic in the device may be different in different locations or regions of the device. For example, a device comprising a flange component, such as (230), (830) or (930), may have a higher wt. % of ceramic in the flange component than in the region of the device that is inserted into the burr hole. Thus, the flange component (230) may have a higher wt. % of ceramic than the stem component (240), and the flange components (830) and (930) may have a higher wt. % of ceramic than the sections of the cylindrical hollow cores (820) or (920) that are inserted in the burr holes. In embodiments, the amount of ceramic present in the region of the device that is inserted into a burr hole is 0.5 to 50 wt. %. In embodiments, the amount of ceramic in the implanted device is greater at the entry point to the burr hole than the amount of ceramic present in the part of the device that is located in the burr hole and surrounded by the diploë. Increased amounts of ceramic in the implanted device in the vicinity of the entry point to the burr hole promotes the formation of hard tissue at the entry point that cannot be depressed. The placement of a device containing high percentages of ceramic in the vicinity of the burr hole entry point helps to prevent the formation of soft tissue in this vicinity, and to prevent the formation of palpable tissue and depressions in the cranium. Lower percentages of ceramic in the implanted device that is surrounded by the diploë helps to create a slightly softer and more porous cancellous bone structure.

E. Fabrication of devices for reconstruction of burr holes

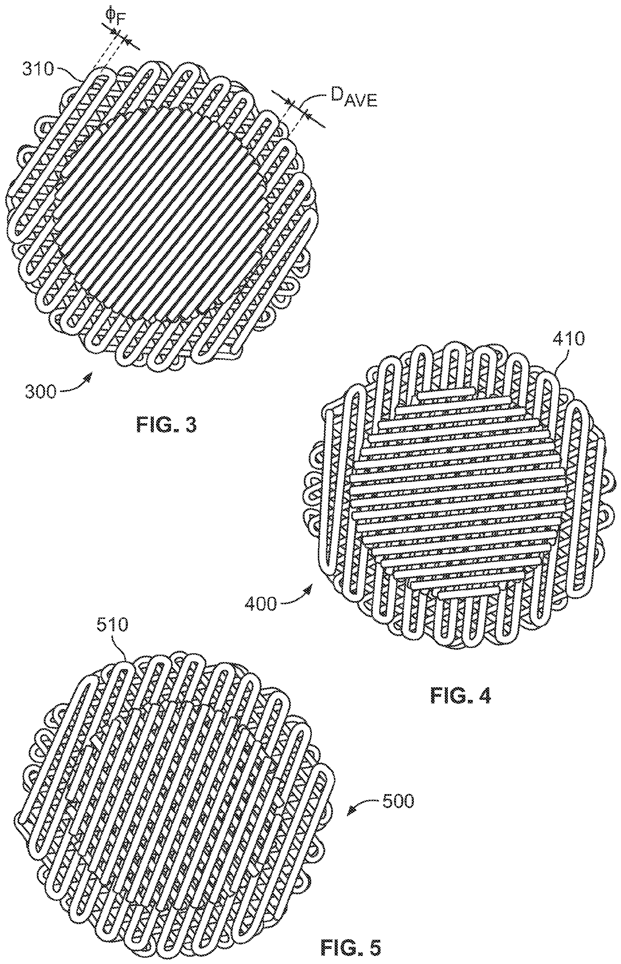

In one example, the devices are prepared by 3D printing. Suitable methods for 3D-printing the devices include fused filament fabrication, fused pellet deposition, melt extrusion deposition, selective laser melting, printing of slurries and solutions using a coagulation bath, and printing using a binding solution and granules of powder. Preferably, the devices are prepared by melt extrusion deposition. The devices depicted in FIGS. 3-7 were manufactured by melt extrusion deposition.

In a typical procedure, the device is prepared by melt extrusion deposition of a resorbable polymer and preferably a blend of a resorbable polymer with a ceramic. A device may be formed, for example, from a blend of P4HB and a ceramic, preferably, β-TCP, using the following procedure. Pellets of P4HB (e.g. Mw of 100-600 kDa) are blended with β-TCP, and compounded prior to 3D printing. Preferably, the amount of β-TCP in the device is 0.5 to 80 wt. %, and more preferably 5 to 50 wt. %. The pellets comprising P4HB and β-TCP may be 3D printed to form the burr hole repair device (e.g. as shown in FIGS. 2A-C) using, for example, an Arburg Freeformer 3D printer, and using the printing parameters shown in Table 1, and a 3D CAM (Computer Aided Design Model) for the burr hole implant. The average diameters of the 3D filaments that are printed are selected based upon the properties of the device desired, including the porosity or fill density (i.e. the number of 3D printed filaments per mm between the contours of the 3D printed device). Preferably, the average filament diameters are 50 to 800 $\mu$m, more preferably 100 to 600 $\mu$m, and even more preferably 150 to 550 $\mu$m.

The print pattern is also selected based on the properties of the device desired. For example, the filaments may be layers printed at 0, 60, and 120 degree angles to each other forming a triangular open pore structure. Or, the filaments may be arranged at other angles to one another, resulting in other geometrical open pore structures including for example, square, quadrilateral, parallelogram, and other shapes whether polygons or not. 3D Printing of the device is highly desirable since it allows precise control of the shape of the device. In one embodiment, the 3D printed device is printed with the shape shown for device (200) wherein the device has a round cap (220) connected to a round stem (240) of a smaller diameter creating a flange (230). The device (200) also has filament elements (210) protruding from the circumference of the stem. The dimensions of the cap, stem and flange are selected according to the dimensions of the burr hole that needs to be filled. The flange is printed to extend at least 2 mm beyond the edge of the burr hole such that the diameter of the printed flange is at least 4 mm larger than the diameter of the burr hole. The stem (240) is printed such that its length in the longitudinal direction ($A_S$) is less that the thickness of the burr hole. Typically a stem (240) with a length of 2 to 7 mm is printed.

The cap (220) is preferably printed so that its thickness in the longitudinal direction (in the direction of the stem) is 1 mm to 5 mm. The filament elements are preferably printed so that they extend outwards 0.1 to 5 mm, and more preferably 1 to 2 mm from the circumference of the stem. The filament elements are preferably printed with diameters of 0.15 to 0.8 mm. The cap preferably has a convex shape. The size of the filament and the print pattern are tailored in order to select a desired porosity of the device. The device may be printed so that the porosity, or filling density, of the device may be the same throughout the device, or it may be different in different regions of the device. For example, the cap (220) of the device may be printed with a different filling density than the stem (240) of the device.

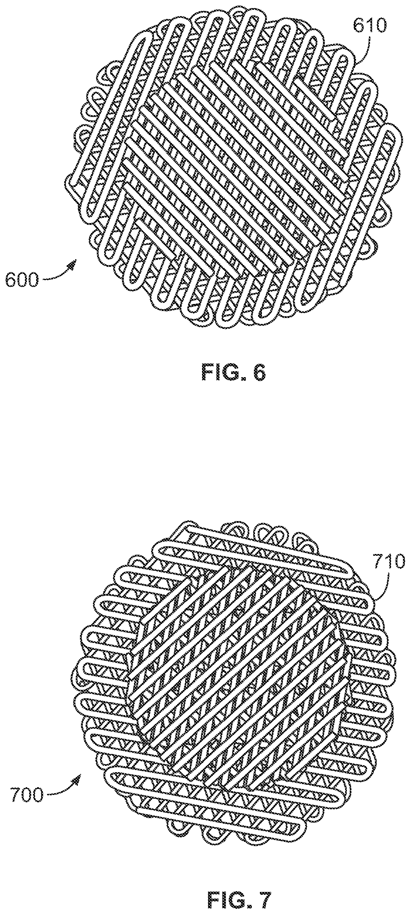

FIGS. 3-7 show devices for filling burr holes that were 3D printed with different filling densities. The device shown in FIG. 3, e.g., has a filling density of 70%. The device shown in FIG. 4 has a filling density of 65%. The device shown in FIG. 5 has a filling density of 55%. The device shown in FIG. 6 has a filling density of 45%, and the device shown in FIG. 7 has a filling density of 40%. As is evident from FIGS. 3-7, the porosity of the device increases as the filling density decreases. The filling density may also be increased in order to eliminate porosity or to make less porous devices. As described in Example 8, herein, the filling density may be varied during the manufacture of the device, for example, to make a solid cap (220) connected to a porous stem (240), or for example to make a device with porosity in both the cap (220) and stem (240), but with different levels of porosity in the cap (220) and stem (240). The device may be 3D printed with a porosity of 0.5 to 80%, but more preferably with a porosity of 30 to 75%. The device may be printed so that the sizes of pores or distances between filaments in the stem (240) and cap (220) are different. For example, the device may be printed so that the distance between filaments in the stem is 0.05 to 2 mm, more preferably 0.075 to 1 mm, and even more preferably 0.1 to 0.6 mm in the longitudinal direction of the stem, and 0.05 to 1 mm, more preferably 0.075 to 0.5 mm, and even more preferably 0.1 to 0.25 mm in the transverse direction of the stem. And, the device may be printed so that the distance between filaments in the cap is 0.01 to 0.5 mm, more preferably 0.025 to 0.2 mm, and even more preferably 0.025 to 0.1 mm in the longitudinal direction of the cap, and 0.01 to 0.2 mm, more preferably 0.01 to 0.1 mm, and even more preferably 0.01 to 0.05 mm in the transverse direction.

With reference to FIG. 3, the device is preferably printed so that the distances between filaments ($D_{AVE}$) is at least 50 $\mu$m, more preferably at least 100 $\mu$m and even more preferably at least 200 $\mu$m, but less than 1 mm.

In embodiments, the filaments are applied in separate or individual layers (e.g., one layer at a time on top of each other, namely, stacked). Within a single layer, each filament can have the same orientation or direction. For example, as shown in FIG. 3, the filaments in each layer extend in the same direction traverse to the stem axis and are generally parallel to one another. Additionally, the distances between filaments 310 are equal. However, in other embodiments (not shown), the distances between filaments within a single layer are varied.

As described herein, in embodiments, layers of filaments are applied, printed or stacked (one on top of the other) to form the device. A second layer of filaments having filaments oriented in a second direction or angle are applied on top of a first layer of filaments having filaments oriented in a first direction or angle. Applying layers of filaments having different orientations creates a crisscross, triangular, or other polygon-like open pore structure when viewed from the top or bottom of the device as shown in, e.g., FIGS. 2B, 2C.

The device may also be printed so that the wt. % of ceramic (e.g. β-TCP) in the filaments located in the stem (240) is different to the wt. % of ceramic in the filaments located in the cap (220). For example, the device may be printed so the filaments in the stem (240) have 0.5 to 50 wt. %, more preferably 5 to 40 wt. %, and even more preferably 10 to 35 wt. % ceramic, and the filaments in the cap (220) have 0.5 to 80 wt. %, more preferably 5 to 50 wt. % ceramic, wherein the amount of ceramic in the filaments in the cap is higher than in the filaments in the stem.

TABLE 1

| Parameters for Melt Extrusion Deposition Printing of P4HB Devices for Reconstruction of Bone Defects | |
| --- | --- |
| Print head temp (°C) | 185 |
| Barrel zone 2 (°C) | 135 |
| Barrel zone 1 (°C) | 100 |
| Build chamber temp (°C) | 10 - 15°C. |
| Screw speed (m/min) | 4 |
| Back pressure (MPa) | 50 |
| Recovery stroke (mm) | 6 |
| Deco speed (mm/s) | 2 |
| Deco stroke (mm) | 4 |
| Discharge nr (%): | 55-75 |
| Filling density (%) | 30-100 |
| Drop ratio | 1.27-1.30 |

The parameters shown in Table 1 may be used to 3D print the devices using P4HB or a blend of P4HB with a ceramic, such as β-TCP. The parameters shown in Table 2 may be used to 3D print the devices using poly(butylene succinate) or copolymer thereof, or a blend of poly(butylene succinate) or copolymer thereof with a ceramic, such as β-TCP.

TABLE 2

| Parameters for Melt Extrusion Deposition Printing of PBS Devices for Reconstruction of Bone Defects | |
| --- | --- |
| Print head temp (°C) | 190-200 |
| Barrel zone 2 (°C) | 150 |
| Barrel zone 1 (°C) | 110 |
| Build chamber temp (°C) | 50 |
| Screw speed (m/min) | 4 |
| Back pressure (MPa) | 50 |
| Recovery stroke (mm) | 6 |
| Deco speed (mm/s) | 2 |
| Deco stroke (mm) | 4 |

(continued)

| Parameters for Melt Extrusion Deposition Printing of PBS Devices for Reconstruction of Bone Defects | |
| --- | --- |
| Discharge nr (%): | 60-75 |
| Filling density (%) | 30-100 |
| Drop ratio | 1.27-1.30 |

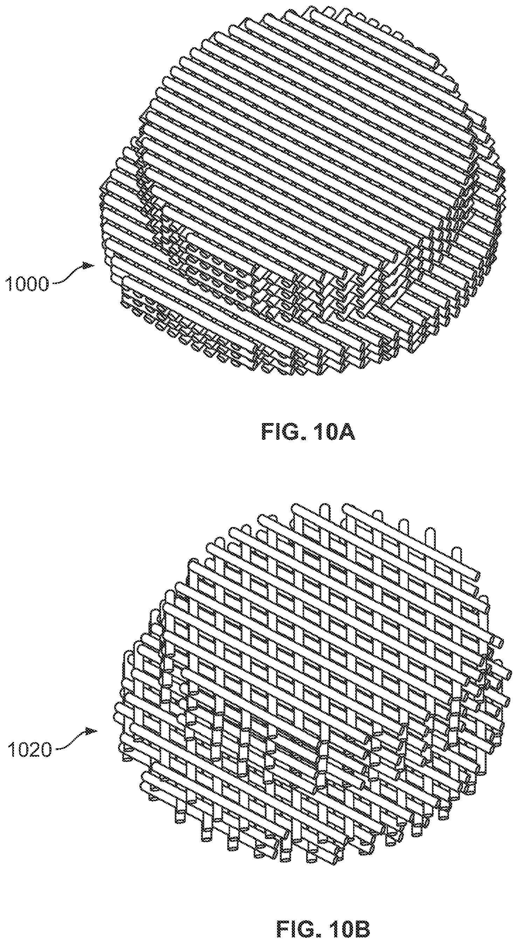

Examples of 3D printed devices to repair bone defects with various open pore structures are shown in FIGS. 10A and 10B. The device shown in FIG. 10A was printed with P4HB containing 20% (w/w) β-TCP, has adjacent layers of filaments oriented at 90 degrees to one another, and has an average pore diameter size of 250 μm. The device shown in FIG. 10B was printed with P4HB, has adjacent layers of fibers oriented at 60 degrees to one another, and has an average pore diameter size of 760 μm. The pictures show a bottom isometric view of the devices with a cylindrical stem connected to a cap with a flange, wherein the stem and cap have an open porous structure.

In the examples shown in FIG. 10A and FIG. 10B, the print or filament orientation angles are 90 and 60 degrees, respectively. However, these angles may be varied to form different shaped open pore structures. Individual layers may be printed at various angles ranging from, e.g., 45-90 degrees.

The number of layers having different orientation or printer angles may also vary. In embodiments, 2-3 different types of layer orientations are applied. However, in other embodiments, 3-5, or more different types or print angles or layer orientations are provided.

Examples of pore shapes arising from the stacked layer arrangement described herein may also vary and include without limitation: triangle, square, trapezoid, parallelogram, diamond, rhomboid, pentagon, or another polygon. In a preferred embodiment, devices to repair bone defects have a triangular open pore structure and are made from P4HB or copolymer thereof, or poly(butylene succinate) or copolymer thereof.

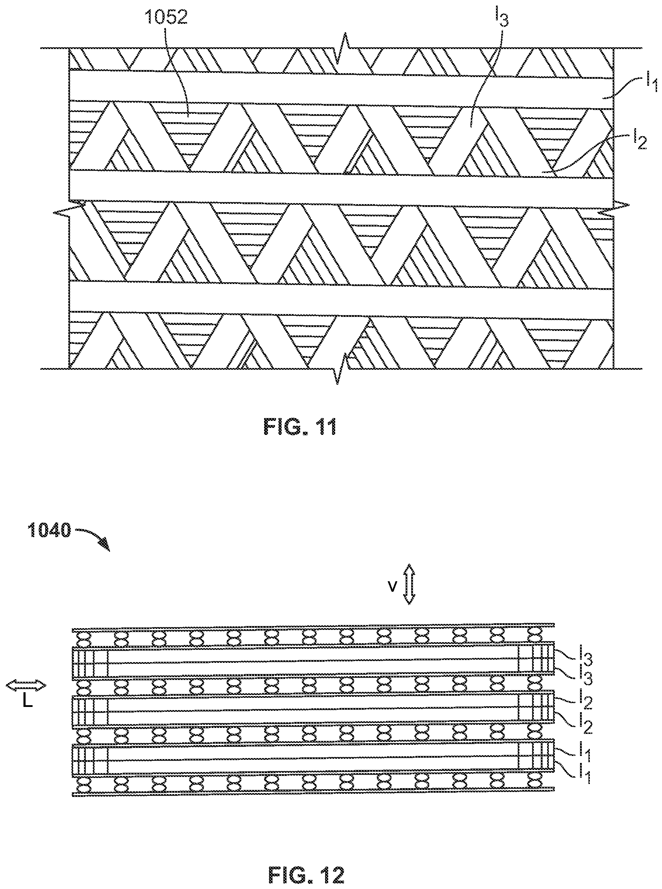

An enlarged portion of an exemplary triangular-shaped pore structure is illustrated in FIG. 11. The triangular open pore structure (1052) is generally defined by stacking layers of filaments such that the filaments (e.g., I1, I2, I3) crisscross. In FIG. 11, there are three types of layers including a first layer having filaments arranged at 0 degrees from horizontal corresponding to filament(s) I1; a second type of layer having filaments orientated at 60 degrees from horizontal corresponding to filament I3, and a third type of layer having filaments arranged at 120 degrees from horizontal corresponding to filament I2. Collectively, the arrangement of the layers having filaments oriented at different angles creates the triangular open pore structure shown in FIG. 11 serving to facilitate tissue ingrowth.

In embodiments, the devices have an elastic modulus between 0.5 MPa and 20 GPa. The cylindrical stems are sized to be inserted in bone defects with flanges that have diameters larger than the diameters of the cylindrical stems.

FIG. 12 shows a side view of a 3D printed structure of the cylindrical stem (1040) of a medical device with an open pore structure (like that shown, e.g., in FIGS. 2-7, 10A and 10B, 11) but where the lateral porosity (L) has been enlarged by once repeating the printing of each filament layer (e.g., I1, I1) before changing the filament orientation or print angle. Repeating a layer of filaments at the same orientation creates an "effective layer" that is double in height (e.g., effective first layer comprises I1 and I1) and increases the lateral porosity (L) of the device.

In embodiments, the lateral porosity (L) is different than the vertical porosity (V). The lateral porosity can be less than or greater than the vertical porosity (V). In embodiments, the lateral porosity is adjusted relative to the vertical porosity by increasing or decreasing the number of repeated layers of filaments.

Repeated printing of layers before changing the print angle may also be used to increase the strength of the device. In the example shown in FIG. 12, two filament layers are printed at an angle of 0 degrees, the print angle is then changed and two filament layers are printed at an angle of 60 degrees before two filament layers are printed at another angle such as e.g., an angle of 120 degrees. The process is then repeated to build up the porous structure to the desired dimensions. In order to create even larger pore sizes, multiple layers (for example, 3, 4, 5, 6, 7, 8, 9, 10 or more) may be printed at the same angle (i.e., repeated) before the print angle is changed. It is to be understood that in accordance with the invention, these angles may be varied to form different shaped open pore structures with two or more filament layers printed at the same angle before the print angle is changed.

The various shaped open pore structures described may be used to prepare any part of the device or all of the device, including the cylindrical stem and the flange. The larger pore sizes, particularly those positioned in the lateral regions of the devices and in the cylindrical stems, are well suited to supporting cell proliferation and tissue in-growth, controlling key cellular processes and pathways, providing nutrition and nutrient transport, for example, to osteoprogenitor cells, and facilitating metabolite discharge, allowing influx of body fluids, and facilitating penetration of the 3D structure by fibrovascular tissue and the development of mature osteons.

In another example, the devices are prepared by molding, and preferably by injection molding. The devices (800) and (900) shown in FIG. 8 and FIG. 9, respectively, may be prepared by molding, and more specifically by injection molding. The device (800) may be prepared by injection molding of component parts (810) and (820) using suitable molds, and assembled by inserting the pin (810) into the cylindrical hollow core (820). The device (900) may also be prepared by injection molding of component parts (910) and (920) using suitable molds, and assembled by threading the pin (910) into the cylindrical hollow core (920). Preferably, the devices (800) and (900) are injection molded from P4HB or copolymer thereof, poly(butylene succinate) or copolymer thereof, or blends of these polymers with ceramics, preferably β-TCP.

The devices may be injection molded from P4HB and copolymers thereof, or from blends of P4HB and copolymers with a ceramic, for example β-TCP, using the following procedure.

The polymer or blend is dried prior to molding to avoid a substantial loss of intrinsic viscosity. Preferably, the polymer or blend is dried so that the moisture content of the molding composition is no greater than 0.5 wt. % as measured gravimetrically, and more preferably no greater than 0.05 wt. %. The polymer or blend may be dried *in vacuo.* In a particularly preferred method, the polymer or blend is dried in a vacuum chamber under a vacuum of at least 10 mbar, more preferably of at least 0.8 mbar, to a moisture content of less than 0.03% by weight. Elevated temperatures below the melting point of the polymer pellets may also be used in the drying process. Alternatively, the polymer may be dried by extraction into a solvent and reprecipitation, or with the use of desiccants. The moisture content of the polymer or blend may be determined using a VaporPro Moisture Analyzer from Arizona Instruments, or similar instrument. Injection molding of the polymer or blend uses controlled processing conditions of temperature, time, speed, and pressure, wherein the dried pellets are melt processed, injected into a mold, cooled, and then molded. A suitable injection molding machine for preparing the device is a hydraulic injection molding machine with an 18 mm screw with four heat zones to melt the polymer or blend. Suitable conditions for injection molding the devices are given in Table 3. As shown in Table 3, the heat zones may be set at: zone 1 150-180°C, zone 2 170-190°C, zone 3 180-220°C, and nozzle 180-220°C. The mold temperature may be set at 3-40°C. The extruder screw speed may be set at 20-400 rpm, and more preferably at 300 rpm. Preferably, the injection pressure is set at 750 psi (5.17 MPa) to 1250 psi (8.62 MPa), and more preferably 850 psi (5.86 MPa) to 1000 psi (6.89 MPa). In an embodiment, the injection speed is set in the range of 5 cm/sec to 20 cm/sec, and more preferably around 10 cm/sec. The injection filling cycle is dependent upon the intrinsic viscosity of the polymer or blend, and may be adjusted as desired by selection of the injection speed, polymer or blend melt temperature, and mold temperature. In an embodiment, the holding pressure is set at 750 psi (5.17 MPa) to 1250 psi (8.62 MPa), and more preferably 850 psi (5.86 MPa) to 1000 psi (6.89 MPa), and the holding time is preferably set at 2 to 8 seconds, and more preferably 3 to 5 seconds. Preferably, the cooling time for the molded devices or components is 60 to 150 seconds, and more preferably 90 to 120 seconds. One skilled in the art will recognize that the process is not limited to the conditions of Table 3 or to hydraulic injection molding machines, and that the moldings of the invention may also be made using electric, mechanical, and hybrid injection molding machines.

TABLE 3

| Typical Settings for Injection Molding P4HB | |
| --- | --- |
| Injection Molding Settings | Range of Values |
| Zone 1 (°C) | 150-200 |
| Zone 2 (°C) | 150-200 |
| Zone 3 (°C) | 150-225 |
| Nozzle (°C) | 150-225 |
| Mold (°C) Fixed Half | 28-35 |
| Mold (°C) Movable Half | 28-35 |
| Injection Pressure (MPa) | 5.8-6.9 |
| Hold Pressure (MPa) | 5.8-6.9 |
| Hold Time (sec) | 5 |
| Cool Time (sec) | 90-120 |

In embodiments, the injection molded devices comprising P4HB or copolymers thereof are annealed. Annealing of the devices increases the crystallinity of the polymer or blend. Preferably, the P4HB injection molded devices are annealed at temperatures preferably of 45-55 °C, but not exceeding 60 °C. In a preferred embodiment, the injection molded devices may be heated in a water bath. One skilled in the art will recognize that the ratio of crystalline content of the injection molded devices may also be tailored by changing the retention time in the mold, the speed of cooling the mold, and by annealing the molded products in a post molding heat cycle.

Devices for closing burr holes, such as (800) and (900), may be injection molded from poly(butylene succinate) and copolymers thereof, and blends of poly(butylene succinate) and copolymers thereof with ceramics, including β-TCP, using the conditions shown in Table 3.

## IV. **METHODS OF IMPLANTING DEVICES TO CLOSE BURR HOLES**

The devices are implanted in the body to repair bone defects, and more specifically, the devices are implanted in the cranium to close burr holes.

Prior to implantation, the devices are sterilized. The devices may be sterilized, for example, by use of ethylene oxide gas, or exposure to gamma-irradiation or electron-beam.

A device of an appropriate size for closure of a burr hole is preferably selected based on the size of the drill bit used to form the burr hole. Or, alternatively, a device of an appropriate size is selected by measurement of the diameter of the burr hole. The diameter of the stem (240) or the cylindrical hollow core (820) or (920) that is inserted in the burr hole is preferably the diameter of the burr hole ±2 mm, or more preferably the diameter of the burr hole ±1 mm. For example, if the diameter of the burr hole is 10 mm, a device with a stem or cylindrical hollow core having a diameter of 10 mm ±2 mm, or more preferably 10 mm ±1 mm is selected.

In embodiments, the diameter of the stem is preferably greater than the diameter of the burr hole by 0.5 - 2 mm such that an interference fit is formed when the stem is located in the burr hole.

The method of implantation of the devices will depend upon the specific design of the device.

Devices comprising a cap (220) and stem (240) with filament elements (240), such as (200) shown in FIGS. 2A-C, may be implanted in a burr hole by inserting the stem (240) in the burr hole until the flange (230) is seated on the outer surface of the cranium. When the device is inserted in the burr hole, the filament elements engage the cancellous bone of the cranium securing the self-locking device in place. In a preferred embodiment, the device (e.g. 200) is coated with autologous blood from the calvarium marrow space prior to insertion in the burr hole. Optionally, the device (200) may also be fixated in place using fibrin glue.

Devices comprising a pin and cylindrical hollow core, such as (800) and (900) shown in FIG. 8 and FIG. 9, respectively, may be implanted in a burr hole by first inserting the cylindrical hollow core (820) or (920) into the burr hole. The cylindrical hollow cores are inserted in the burr holes until the flanges (830) and (930) abut the outer surface of the cranium. The cylindrical hollow cores (820) and (920) may be coated with autologous blood from the calvarium marrow space prior to implantation. Optionally, the cylindrical hollow cores (820) and (920) may be fixated in place using fibrin glue.

After insertion of the cylindrical hollow cores, e.g. (820) or (920), the pins (810) and (910) respectively, may be inserted into the cylindrical hollow cores. The pin (810) may simply be pushed into the cylindrical hollow core (820) until it is seated in place, and expansion of the cylindrical hollow core by the pin (810) secures the device in the burr hole. In embodiments, an interference fit is created.

The pin stop (840) prevents the pin (810) from traveling through the burr hole and exiting into the space between the inner cranium surface and the meninges, and provides resistance for the rivet push pin (810) to be compressed and engaged in the cylindrical hollow core (820). The pin (910) may be screwed into the cylindrical hollow core (920) as shown in FIG. 9A by inserting a suitable tool into the hexagonal socket and rotating the pin until the pin is fully inserted in the cylindrical hollow core (920).

Alternatively, the device (800) may be implanted in one step, wherein the pin (810) is preloaded in the cylindrical hollow core (820) but not completely inserted inside the hollow core, and the assembly of the pin and cylindrical hollow core is implanted. The assembly is pushed into the burr hole until the flange (830) of the cylindrical hollow core contacts the outer surface of the cranium. The pin (810) is then pushed down into the cylindrical hollow core (820) causing expansion of the cylindrical hollow core and securing the device in the burr hole. The device (800) may be coated with autologous blood from the calvarium marrow space prior to implantation. Optionally, fibrin glue may be used to help fixate the device (800) in the burr hole.

After insertion of the devices (e.g. (200), (800) or (900)) in the burr hole, the scalp is preferably closed with a resorbable suture, such as Vicryl size 3/0, and the skin closed with a permanent suture, such as Prolene size 3/0.

The devices are used to close burr holes that are made during the treatment of chronic subdural hematoma, epidural hematoma, as well as in other types of neurosurgery.

The present invention will be further understood by reference to the following non-limited examples.

EXAMPLES

Example 1: Porous implant for burr hole closure made by 3D printing of P4HB comprising β-TCP

Porous implants for closure of a burr hole bone defect were made from compounded pellets of P4HB (Mw 380 kDa) containing 5, 20, and 40 wt. % of beta-tricalcium phosphate (β-TCP). The implants were prepared by 3D printing melt extrusion deposition using the print parameters shown in Table 1 and an Arburg Freeformer 3D printer. The design of the implant (200) is shown in FIG. 2. The printed filaments of the implant had an average diameter of 250 μm. Layers of the filament were crisscrossed at 0, 60, and 120-degree angles. A triangular open pore structure was formed using a lay down pattern with layers oriented at 0/60/120° to each other. As shown in FIGS. 2A-2C, the implants (200) had a shape comprising two portions. A first portion which was a cap (220) with a flange (230), and a second portion which was a stem (240) (or boss). The cap was sized so that the flange (230) would extend beyond the contour of the defect on the outside of the bone defect to ensure that the stem remained in the bone defect. The stem (240) was sized so that it would remain within the burr hole upon implantation. The diameter of the flange was 10-25 mm, and the diameter of the stem was 7-19 mm. The implants had a porosity of 30-75% with average distances between filaments of 200 μm to allow tissue in-growth.

Example 2: Porous implant for burr hole closure made by 3D printing of P4HB with 70% filling density

The method described in Example 1 was repeated, except that the implant was only made from P4HB, and not from β-TCP compounded with P4HB. The implant was produced using the same print parameters listed in Table 1. The implant was produced with P4HB filaments having average diameters of 285 μm, which

were deposited layer-by-layer using melt extrusion deposition. The average distance between filaments ($D_{AVE}$) was 200 μm. The implant, produced according to this method and shown in FIG. 3 had a filling density of 70% and a drop ratio of 1.3.

Example 3: Porous implant for burr hole closure made by 3D printing of P4HB with 65% filling density

The method described in Example 2 was repeated, except the filling density shown in Table 1 was changed from 70% to 65%. The average distance between filaments of the implant was 250 μm. The implant, produced according to this method is shown in FIG. 4.

Example 4: Porous implant for burr hole closure made by 3D printing of P4HB with 55% filling density

The method described in Example 2 was repeated, except the filling density shown in Table 1 was changed from 70% to 55%. The average distance between filaments of the implant was 350 μm. The implant, produced according to this method is shown in FIG. 5.

Example 5: Porous implant for burr hole closure made by 3D printing of P4HB with 45% filling density

The method described in Example 2 was repeated, except the filling density shown in Table 1 was changed from 70% to 45%. The average distance between filaments of the implant was 500 μm. The implant, produced according to this method is shown in FIG. 6.

Example 6: Porous implant for burr hole closure made by 3D printing of P4HB with 40% filling density

The method described in Example 2 was repeated, except the filling density shown in Table 1 was changed from 70% to 40%, and the average distance between filaments of the implant was 575 μm. The implant, produced according to this method is shown in FIG. 7.

Example 7: Porous implant for burr hole closure made by 3D printing of poly(butylene succinate) with filling densities of 35%, 40% and 45%

The method described in Example 1 was repeated, except that the implants were made from poly(butylene succinate) (Mw 177 kDa) instead of P4HB, and the filling densities were 35% 40% and 45%. The device was printed using the parameters shown in Table 1 with average filament diameters of 245 μm. The filaments were deposited layer-by-layer using melt extrusion deposition 3D printing. The filament layers were crisscrossed at 0, 60 and 120 degree angles. A lay down pattern of layers oriented at 0/60/120° to each other was used to form the triangular open pore structure.

Example 8: Porous implant for burr hole closure made by 3D printing of P4HB and β-TCP having a hard cap

The method described in Example 1 was repeated, except that the print density of the cap component of the implant was increased to produce a solid cap with no interconnected pores.

## Claims

1. A device (100,200,300,400,500,600,700) to repair a bone defect, wherein the device comprises a stem (110,240) having a circumference, the stem being connected to a cap (120,220) with a flange (190,230), wherein the device is porous, **characterised in that** the device further comprises bristles (210,310,410,510,610,710) emanating from the circumference of the stem.

2. The device of claim 1, wherein the device has pores in the cap and stem, and wherein the dimensions of the pores in the stem are larger than the pores in the cap.

3. The device of claim 1, wherein the device comprises a ceramic, and wherein the concentration of ceramic in the stem is lower than the concentration of ceramic in the flange.

4. The device of claim 2, wherein the average pore dimensions in the stem are 0.05 to 2 mm, and the average pore dimensions in the cap are 0.01 to 0.5 mm.

5. The device of claims 1 and 4, wherein the device comprises a resorbable polymer.

6. The device of claim 5, wherein the resorbable polymer is poly-4-hydroxybutyrate or copolymer thereof, or poly(butylene succinate) or copolymer thereof.

7. The device of claim 1, wherein the bone defect is a burr hole (130) in the cranium (140), and the device is sized to plug the burr hole.

8. The device of claim 7, wherein stem of the device is sized to create an interference fit with the burr hole.

9. The device of claim 1, wherein the bristles extend from the circumference of the stem at least 1 mm.

10. The device of any one of claim 1-9, wherein the stem and cap are formed by stacking a plurality of layers of filaments.

## Patentansprüche

1. Vorrichtung (100,200,300,400,500,600,700) zum Reparieren eines Knochendefekts, wobei die Vorrichtung einen Schaft (110,240) mit einem Umfang umfasst, wobei der Schaft mit einer Kappe (120,220) mit einem Flansch (190,230) verbunden ist, wobei die Vorrichtung porös ist, **dadurch gekennzeichnet, dass** die Vorrichtung weiter Borsten (210,310,410,510,610,710) umfasst, die von dem Umfang des Schafts ausgehen.

2. Vorrichtung nach Anspruch 1, wobei die Vorrichtung Poren in der Kappe und in dem Schaft aufweist, und wobei die Größen der Poren in dem Schaft größer sind als die Poren in der Kappe.

3. Vorrichtung nach Anspruch 1, wobei die Vorrichtung eine Keramik umfasst, und wobei die Konzentration der Keramik in dem Schaft geringer ist als die Konzentration der Keramik in dem Flansch.

4. Vorrichtung nach Anspruch 2, wobei die durchschnittliche Porengröße in dem Schaft 0,05 bis 2 mm beträgt und die durchschnittliche Porengröße in der Kappe 0,01 bis 0,5 mm beträgt.

5. Vorrichtung nach den Ansprüchen 1 und 4, wobei die Vorrichtung ein resorbierbares Polymer umfasst.

6. Vorrichtung nach Anspruch 5, wobei das resorbierbare Polymer Poly-4-hydroxybutyrat oder ein Copolymer davon ist, oder Poly(butylensuccinat) oder ein Copolymer davon.

7. Vorrichtung nach Anspruch 1, wobei der Knochendefekt ein Gratloch (130) in dem Schädel (140) ist, und die Vorrichtung so bemessen ist, dass sie das Gratloch verschließt.

8. Vorrichtung nach Anspruch 7, wobei der Schaft der Vorrichtung so bemessen ist, dass er eine Presspassung mit dem Gratloch bildet.

9. Vorrichtung nach Anspruch 1, wobei die Borsten sich mindestens 1 mm von dem Umfang des Schafts erstrecken.

10. Vorrichtung nach einem der Ansprüche 1-9, wobei der Schaft und die Kappe durch Stapeln einer Vielzahl von Lagen von Filamenten gebildet sind.

## Revendications

1. Dispositif (100, 200, 300, 400, 500, 600, 700) pour réparer un défaut osseux, dans lequel le dispositif comprend une tige (110, 240) présentant une circonférence, la tige étant reliée à un bouchon (120, 220) avec une bride (190, 230), dans lequel le dispositif est poreux, **caractérisé en ce que** le dispositif comprend en outre des poils (210, 310, 410, 510, 610, 710) émanant de la circonférence de la tige.

2. Dispositif selon la revendication 1, dans lequel le dispositif présente des pores dans le bouchon et la tige, et dans lequel les dimensions des pores dans la tige sont plus larges que celles des pores dans le bouchon.

3. Dispositif selon la revendication 1, dans lequel le dispositif comprend une céramique, et dans lequel la concentration de céramique dans la tige est inférieure à la concentration de céramique dans la bride.

4. Dispositif selon la revendication 2, dans lequel les dimensions moyennes des pores dans la tige sont de 0,05 à 2 mm, et les dimensions moyennes des pores dans le bouchon sont de 0,01 à 0,5 mm.

5. Dispositif selon les revendications 1 et 4, dans lequel le dispositif comprend un polymère résorbable.

6. Dispositif selon la revendication 5, dans lequel le polymère résorbable est du poly-4-hydroxybutyrate ou un copolymère de celui-ci, ou du poly(succinate de butyle) ou un copolymère de celui-ci.

7. Dispositif selon la revendication 1, dans lequel le défaut osseux est un orifice de trépan (130) dans le crâne (140), et le dispositif est conçu pour boucher l'orifice de trépan.

8. Dispositif selon la revendication 7, dans lequel la tige du dispositif est conçue pour créer un ajustement serré avec l'orifice de trépan.

9. Dispositif selon la revendication 1, dans lequel les poils s'étendent d'au moins 1 mm depuis la circonférence de la tige.

10. Dispositif selon l'une quelconque des revendications 1 à 9, dans lequel la tige et le bouchon sont formés par empilement d'une pluralité de couches de filaments.

**FIG. 1**

EP 4 076 288 B1

FIG. 2A

FIG. 2B

FIG. 2C

EP 4 076 288 B1

FIG. 3

FIG. 4

FIG. 5

EP 4 076 288 B1

FIG. 6

FIG. 7

28

**FIG. 8A**

**FIG. 8B**

**FIG. 8C**

EP 4 076 288 B1

900

910

920

FIG. 9C

900

910

9C

920

9C

FIG. 9B

920

950

FIG. 9E

900

910

920

FIG. 9A

960

930

940

920

FIG. 9D

FIG. 10A

FIG. 10B

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20070083268 A, Teoh
- US 6350284 B, Törmälä
- US 2015150681 A, Ricci

### Non-patent literature cited in the description

- The efficacy of titanium burr hole cover for reconstruction of skull defect after burr hole trephination of chronic subdural hematoma. *Korean J Neurotrauma,* 2014, vol. 10 (2), 76-81
- **KUBOTA et al.** Long-term follow-up for ossification of autologous bone plug and skin sinking after periosteum-preserved burr hole surgery. *Surgical Neurology International,* 2017, vol. 8, 204
- **SCHANTZ et al.** Cranioplasty after trephination using a novel biodegradable burr hole cover: technical case report. *Neurosurgery,* 2006, vol. 58 (1
- **GORBET et al.** *Biomaterials,* 2005, vol. 26, 6811-6817
- **WILLIAMS et al.** Poly-4-hydroxybutyrate (P4HB): a new generation of resorbable medical devices for tissue repair and regeneration. *Biomed. Tech.,* 2013, vol. 58 (5), 439-452